# EUROPEAN PATENT APPLICATION

(11) **EP 4 632 764 A1**
(43) Date of publication of application: **15.10.2025**
(21) Application number: 23900709.9
(22) Date of filing: 06.12.2023
(51) Int. Cl.: G16H 80/00, G06Q 50/10

(54) **REMOTE MEDICAL EXAMINATION DEVICE FOR ANIMALS, AND DEVICE FOR COMMISSIONING OPERATION FOR ISSUEING DIRECT MAIL**

(30) Priority: 06.12.2022 JP 2022195226; 06.12.2022 JP 2022195227
(71) Applicant: PETCOMMUNICATIONS CO., LTD., Osaka-shi, Osaka 541-0048 (JP)
(72) Inventor: MINAMINO, Norio, Osaka-shi, Osaka 541-0048 (JP)
(74) Representative: Forresters IP LLP
(86) International application number: PCT/JP2023/043737
(87) International publication number: WO 2024/122597

(57) **Abstract**

A first physician in a first veterinary hospital A transmits a second medical chart not including names and addresses of animal owners from a first control processing device 1 to a second control processing device 2 of a second physician in a second veterinary hospital D via a server **3.** The second physician can create a medical diagnostic report 15 by performing medical diagnosis on the basis of the second medical chart **12.** The first physician obtains the medical diagnostic report 15 to perform medical diagnosis and medical treatment. The second physician can additionally request additional examination data 16 to the first physician to create a medical diagnostic report 15 on the basis of the second medical chart 12 and the additional examination data **16.** Tasks of issuing direct mail DM can be commissioned.

## Description

### Technical Field

The present invention relates to a remote medical examination device for animals that allows physicians, who are veterinarians at respective veterinary hospitals, to exchange medical information for medical care using a communication line (network) such as the Internet, and the present invention further relates to a medical examination device for animals that can commission the operation (task) for issuing direct mail.

### Background Art

Veterinarians in veterinary hospitals perform "medical checkup" for checking the bodies of reared animals including pet animals (i.e. pets), such as dogs and cats, or other animals to determine their medical conditions, perform "medical diagnosis" on the basis of the results of the medical checkup and examination data to determine a medical treatment policy, and the veterinarians performs "medical treatment" to heal diseases and alleviate symptoms. In this specification, medical care may refer to any one or more of the medical checkup, the medical diagnosis, and the medical treatment.

### Prior Art

In recent years, veterinarians in veterinary hospitals have played important roles, and the information handled by veterinary hospitals has been increasing year by year. In the field of medical care for animals, veterinarians are so-called general physicians who supposedly provide general medical care for many kinds of animals. However, it cannot be ignored that the veterinarians have their skilled and unskilled areas depending on the medical care fields. Therefore, it may not be possible to provide smooth, rapid and advanced medical care with general physicians alone. In order to solve this problem, some countries have established a medical specialist system for each medical specialty of veterinarians.

### Citation List

### Patent Literature

PTL1: Japanese Patent Application Laid-Open No. 2022-44667

### Summary of Invention

### Technical Problem

Regarding the interactions between the animal owners and veterinarians, a support system has been proposed that improves the quality of animal body images captured by owners using cameras and the quality of sounds sent to each other, for accurate remote medical checkup of pets and other animals (Patent Literature 1). With this conventional technology, for example, the medical care in a medical field where a certain general physician who is a veterinarian is not skilled cannot be supplemented by the medical knowledge of a medical specialist who is a different veterinarian that is skilled in the aforementioned medical field. As a result, there is a need for more advanced medical care for animals.

It is an object of the present invention to provide a remote medical examination device for animals that allows smooth, rapid, and advanced medical care for animals.

### Solution to Problem

For the sake of brevity, reference signs of constituent elements are provided such that A, B, and/or C may be collectively referred to as A, and D and/or E may be collectively referred to as D, and individual suffixes of numerals such as a and b may be omitted so as to collectively express 1a, 1b, and/or 1c as 1, 2d and/or 2e as 2, 5a, 5b, and/or 5c; 6d and/or 6e as 5 and 6. In the description of each aspect of the present invention described below, the same terms designate the same constituent elements, and for each term that appears more than once, a prepositional phrase "above-described" or "said" is omitted in the second and subsequent appearance.

The present invention relates to a remote medical examination device for animals, the remote medical examination device including:
(a) a first control processing device 1a, 1b, 1c for a first physician installed at a first veterinary hospital A, B, C;
(b) a second control processing device 2d, 2e for a second physician installed at a second veterinary hospital D, E; and
(c) a server 3 connected to the first control processing device 1 and the second control processing device 2 via communication lines 5a, 5b, 5c; 6d, 6e, wherein:
(d) the first control processing device 1 includes
   (d1) a first memory 10 that stores
      a first medical chart 11 provided for each of one or more animals to be given medical care, the first medical chart 11 including owner information about an owner of each animal, identification information about each animal, medical data by the first physician, and examination data including an image,
      a second medical chart 12 created from the first medical chart 11 with at least part of the owner information excluded, and
      a master application program 14 for creating and outputting the first medical chart 11 and the second medical chart 12,
   (d2) a first processing circuit 17 that executes the master application program 14 to create the first medical chart 11 and the second medical chart 12 and store them in the first memory 10, and
   (d3) a first control communication circuit 18 that
      outputs, to the server 3, a request signal 44 about the animal for which medical diagnosis is requested to the second physician in the second veterinary hospital D selected by the first physician, the second medical chart 12 of the animal for which the medical diagnosis is requested, and the master application program 14,
      receives from the server 3 an acceptance signal 46 and a medical diagnostic report 15 by the second physician about the animal for which the medical diagnosis is requested and stores them in the first memory 10;
(e) the server 3 includes
   (e1) a server control communication circuit 38 that is connected to the first control communication circuit 18 and the second control processing device 2 via the communication lines 5, 6,
   (e2) a server memory 30, and
   (e3) a server processing circuit 37 that
      stores, in the server memory 30, the request signal 44, the second medical chart 12, and the master application program 14 from the first control communication circuit 18, which have been received by the server control communication circuit 38, transmits them to the second control processing device 2 by the server control communication circuit 38,
      stores, in the server memory 30, the acceptance signal 46 and the medical diagnostic report 15 from the second control processing device 2, which have been received by the server control communication circuit 38, and transmits them to the first control communication circuit 18 by the server control communication circuit 38; and
(f) the second control processing device 2 includes
   (f1) a second control communication circuit 28 that receives the request signal 44 from the server control communication circuit 38 and, when accepting the medical care request for each animal, transmits the acceptance signal 46 for each animal to the server 3,
   (f2) a second memory 20 that stores the second medical chart 12 and the master application program 14 downloaded from the server 3 for each animal corresponding to the acceptance signal 46, and
   (f3) a second processing circuit 27 that executes the master application program 14 stored in the second memory 20 to create the medical diagnostic report 15 based on the second medical chart 12 by the second physician, and
   (f4) the second control communication circuit 28 transmits the medical diagnostic report 15 from the second processing circuit 27 to the server 3.

The present invention relates to a remote medical examination method for animals, the remote medical examination method characterized in that:
(a) the remote medical examination device 100 for animals described above is prepared;
(b) the first physician in the first veterinary hospital A selects one or more second physicians in one or more second veterinary hospitals D and an animal for which medical diagnosis is requested, and transmits the second medical chart 12 of the animal for which medical diagnosis is requested and the master application program 14, which have been stored in the first memory 10 of the first control processing device 1, for the selected second veterinary hospital D by the first control communication circuit 18;
(c) the second physician in the second veterinary hospital D accepts the requested medical diagnosis of the animal in the second control processing device 2, creates the medical diagnostic report 15 based on the second medical chart 12 of the animal for which the medical diagnosis is requested, and transmits the medical diagnostic report 15 for the first veterinary hospital A by the second control communication circuit 28; and
(d) the first physician in the first veterinary hospital A determines a medical treatment policy on the basis of the medical diagnostic report 15 that has been created by the second physician and that has been downloaded to the first control processing device 1.

The present invention relates to a remote medical examination device for animals, the remote medical examination device including:
(a) a first control processing device 1a, 1b, 1c for a first physician installed at a first veterinary hospital A, B, C;
(b) a second control processing device 2d, 2e for a second physician installed at a second veterinary hospital D, E; and
(c) a server 3 connected to the first control processing device 1 and the second control processing device 2 via communication lines 5a, 5b, 5c; 6d, 6e, wherein:
(d) the first control processing device 1 includes
   (d1) a first memory 10 that stores
      a first medical chart 11 provided for each of one or more animals to be given medical care, the first medical chart 11 including owner information about an owner of each animal, identification information about each animal, medical data by the first physician, and examination data including an image,
      a second medical chart 12 created from the first medical chart 11 with at least part of the owner information excluded,
      additional examination data 16 added by the first physician in response to an additional request signal 47, and
      a master application program 14 for creating and outputting the first medical chart 11, the second medical chart 12, and the additional examination data 16,
   (d2) a first processing circuit 17 that executes the master application program 14 to create the first medical chart 11, the second medical chart 12, and the additional examination data 16 and store them in the first memory 10, and
   (d3) a first control communication circuit 18 that
      outputs, to the server 3, a request signal 44 about the animal for which medical diagnosis is requested to the second physician in the second veterinary hospital D selected by the first physician, the second medical chart 12 of the animal for which the medical diagnosis is requested, the master application program 14, and the additional examination data 16 corresponding to the additional request signal 47,
      receives from the server 3 an acceptance signal 46 and a medical diagnostic report 15 by the second physician about the animal for which the medical diagnosis is requested and stores them in the first memory 10;
(e) the server 3 includes
   (e1) a server control communication circuit 38 that is connected to the first control communication circuit 18 and the second control processing device 2 via the communication lines 5, 6,
   (e2) a server memory 30, and
   (e3) a server processing circuit 37 that
      stores, in the server memory 30, the request signal 44, the second medical chart 12, the additional examination data 16, and the master application program 14 from the first control communication circuit 18, which have been received by the server control communication circuit 38, transmits them to the second control processing device 2 by the server control communication circuit 38,
      stores, in the server memory 30, the acceptance signal 46, the additional request signal 47, and the medical diagnostic report 15 from the second control processing device 2, which have been received by the server control communication circuit 38, and transmits them to the first control communication circuit 18 by the server control communication circuit 38; and
(f) the second control processing device 2 includes
   (f1) a second control communication circuit 28 that receives the request signal 44 from the server control communication circuit 38 and, when accepting the medical care request for each animal, transmits the acceptance signal 46 for each animal to the server control communication circuit 38,
   (f2) a second memory 20 that stores the second medical chart 12, the additional examination data 16, and the master application program 14 downloaded from the server 3 for each animal corresponding to the acceptance signal 46, and
   (f3) a second processing circuit 27 that executes the master application program 14 stored in the second memory 20 to output the additional request signal 47 that requests the examination data not included in the second medical chart 12 as the additional examination data 16 and to create the medical diagnostic report 15 based on the additional examination data 16 by the second physician, and
   (f4) the second control communication circuit 28 transmits the additional request signal 47 and the medical diagnostic report 15 from the second processing circuit 27 to the server 3.

The present invention relates to a remote medical examination method for animals, the remote medical examination method characterized in that:
(a) the remote medical examination device 100 for animals described above is prepared;
(b) the first physician in the first veterinary hospital A selects one or more second physicians in one or more second veterinary hospitals D and an animal for which medical diagnosis is requested, and transmits the second medical chart 12 of the animal for which medical diagnosis is requested and the master application program 14, which have been stored in the first memory 10 of the first control processing device 1, for the selected second veterinary hospital D by the first control communication circuit 18;
(C) the second physician in the second veterinary hospital D accepts the requested medical diagnosis of the animal in the second control processing device 2, outputs, by the second processing circuit 27, an additional request signal 47 for requesting examination data not included in the second medical chart 12 of the animal, for which medical diagnosis is requested, as the additional examination data 16 to the first physician, and transmits the additional request signal 47 by the second control communication circuit 28;
(d) the first physician in the first veterinary hospital A creates the additional examination data 16 in response to the additional request signal 47 and transmits it for the selected second veterinary hospital D by the first control communication circuit 18;
(e) the second physician in the second veterinary hospital D creates the medical diagnostic report 15 based on the additional examination data 16 in the second control processing device 2 and transmits the medical diagnostic report 15 for the first veterinary hospital A by the second control communication circuit 28; and
(f) the first physician in the first veterinary hospital A determines a medical treatment policy on the basis of the medical diagnostic report 15 that has been created by the second physician and that has been downloaded to the first control processing device 1.

The present invention relates to a remote medical examination device for animals, the remote medical examination device including:
(a) a first control processing device 1a, 1b, 1c for a first physician installed at a first veterinary hospital A, B, C;
(b) a second control processing device 2d, 2e for a second physician installed at a second veterinary hospital D, E; and
(c) a server 3 connected to the first control processing device 1 and the second control processing device 2 via communication lines 5a, 5b, 5c; 6d, 6e, wherein:
(d) the first control processing device 1 includes
   (d1) a first memory 10 that stores
      a first medical chart 11 provided for each of one or more animals to be given medical care, the first medical chart 11 including owner information about an owner of each animal, identification information about each animal, medical data by the first physician, and examination data including an image,
      a second medical chart 12 created from the first medical chart 11 with at least part of the owner information excluded,
      a third medical chart 13 including additional examination data 16 added to the second medical chart 12 by the first physician in response to an additional request signal 47, and
      a master application program 14 for creating and outputting the first to third medical charts 11 to 13,
   (d2) a first processing circuit 17 that executes the master application program 14 to create the first to third medical charts 11 to 13 and store them in the first memory 10, and
   (d3) a first control communication circuit 18 that
      outputs, to the server 3, a request signal 44 about the animal for which medical diagnosis is requested to the second physician in the second veterinary hospital D selected by the first physician, the second medical chart 12 of the animal for which the medical diagnosis is requested, the master application program 14, and the third medical chart 13 corresponding to the additional request signal 47,
      receives from the server 3 an acceptance signal 46 and a medical diagnostic report 15 by the second physician about the animal for which the medical diagnosis is requested and stores them in the first memory 10;
(e) the server 3 includes
   (e1) a server control communication circuit 38 that is connected to the first control communication circuit 18 and the second control processing device 2 via the communication lines 5, 6,
   (e2) a server memory 30, and
   (e3) a server processing circuit 37 that
      stores, in the server memory 30, the request signal 44, the second medical chart 12, the third medical chart 13, and the master application program 14 from the first control communication circuit 18, which have been received by the server control communication circuit 38, transmits them to the second control processing device 2 by the server control communication circuit 38,
      stores, in the server memory 30, the acceptance signal 46, the additional request signal 47, and the medical diagnostic report 15 from the second control processing device 2, which have been received by the server control communication circuit 38, and transmits them to the first control communication circuit 18 by the server control communication circuit 38; and
(f) the second control processing device 2 includes
   (f1) a second control communication circuit 28 that receives the request signal 44 from the server control communication circuit 38 and, when accepting the medical care request for each animal, transmits the acceptance signal 46 for each animal to the server control communication circuit 38,
   (f2) a second memory 20 that stores the second medical chart 12, the third medical chart 13, and the master application program 14 downloaded from the server 3 for each animal corresponding to the acceptance signal 46, and
   (f3) a second processing circuit 27 that executes the master application program 14 stored in the second memory 20 to output the additional request signal 47 for requesting examination data not included in the second medical chart 12 as the additional examination data 16 and to create the medical diagnostic report 15 based on the third medical chart 13 by the second physician, and
   (f4) the second control communication circuit 28 transmits the additional request signal 47 and the medical diagnostic report 15 from the second processing circuit 27 to the server 3.

The present invention relates to a remote medical examination method for animals, the remote medical examination method characterized in that:
(a) the remote medical examination device 100 for animals described above is prepared;
(b) the first physician in the first veterinary hospital A selects one or more second physicians in one or more second veterinary hospitals D and the animal for which medical diagnosis is requested, and transmits the second medical chart 12 of the animal for which medical diagnosis is requested and the master application program 14, which have been stored in the first memory 10 of the first control processing device 1, for the selected second veterinary hospital D by the first control communication circuit 18;
(c) the second physician in the second veterinary hospital D accepts the requested medical diagnosis of the animal in the second control processing device 2, outputs, by the second processing circuit 27, an additional request signal 47 for requesting examination data not included in the second medical chart 12 of the animal, for which medical diagnosis is requested, as the additional examination data 16 to the first physician, and transmits the additional request signal 47 by the second control communication circuit 28;
(d) the first physician in the first veterinary hospital A creates the additional examination data 16 in response to the additional request signal 47 and transmits the third medical chart 13 including the created additional examination data 16 in the second medical chart 12 for the selected second veterinary hospital D by the first control communication circuit 18;
(e) the second physician in the second veterinary hospital D creates the medical diagnostic report 15 based on the third medical chart 13 in the second control processing device 2 and transmits the medical diagnostic report 15 for the first veterinary hospital A by the second control communication circuit 28; and
(f) the first physician in the first veterinary hospital A determines a medical treatment policy on the basis of the medical diagnostic report 15 that has been created by the second physician and that has been downloaded to the first control processing device 1.

In the present invention,
the owner information includes at least one of a name and an address of the owner,
the identification information about the animal includes at least one character of a numeral and a symbol to identify the animal,
the medical data includes data about at least one of vaccine, filaria prevention, and flea prevention for medical checkup, medical diagnosis, and medical treatment,
the examination data including an image includes an X-ray photograph of the body of the animal, data on at least one of blood test and blood chemistry screening test, and
the second medical chart 12 does not include the name and address of the owner.

The present invention relates to a computer program for causing a computer to function as the remote medical examination device for animals described above.

The present invention relates to a recording medium that stores the computer program described above.

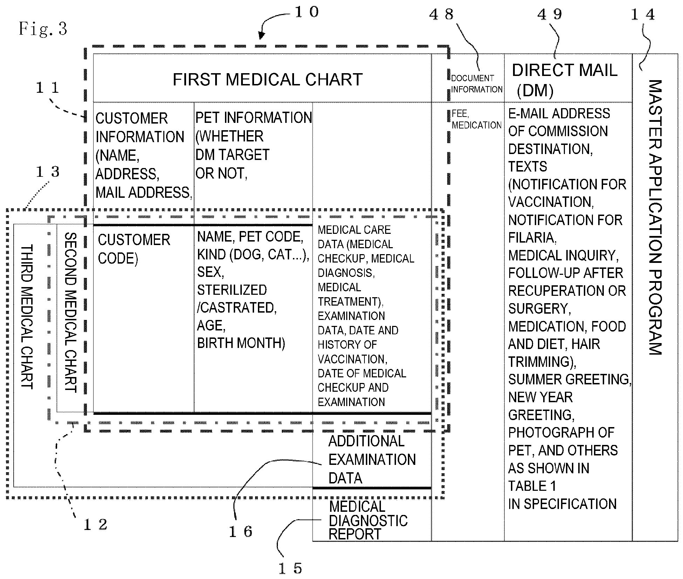

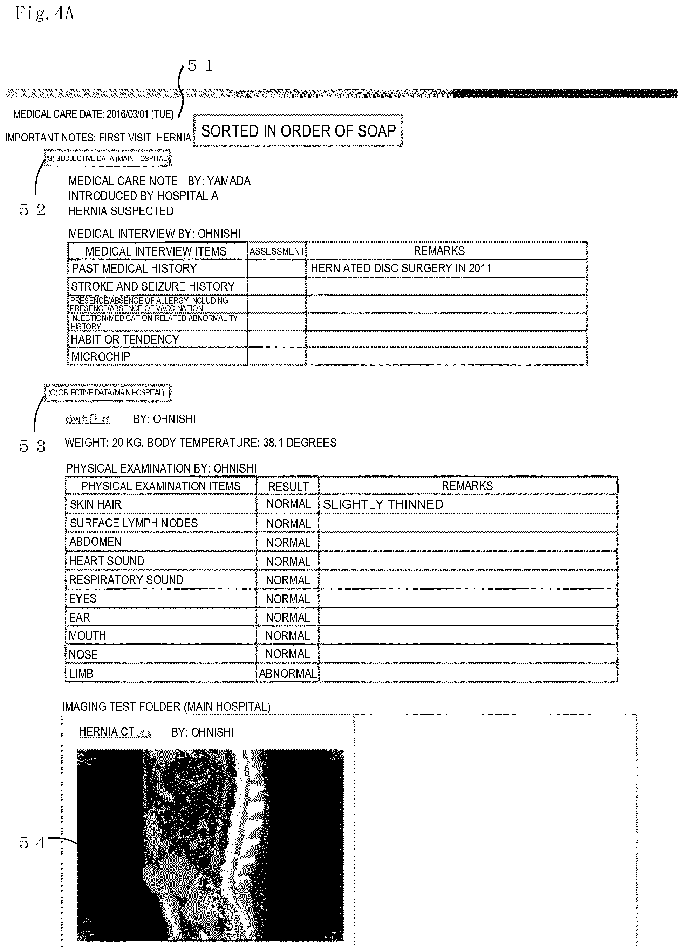

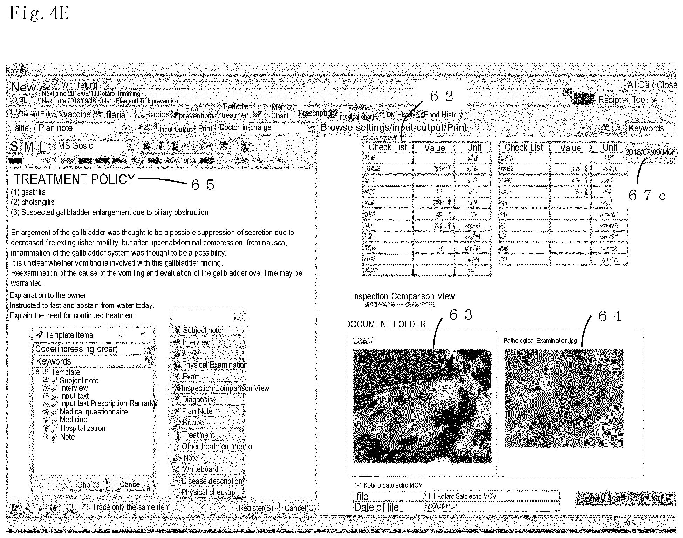

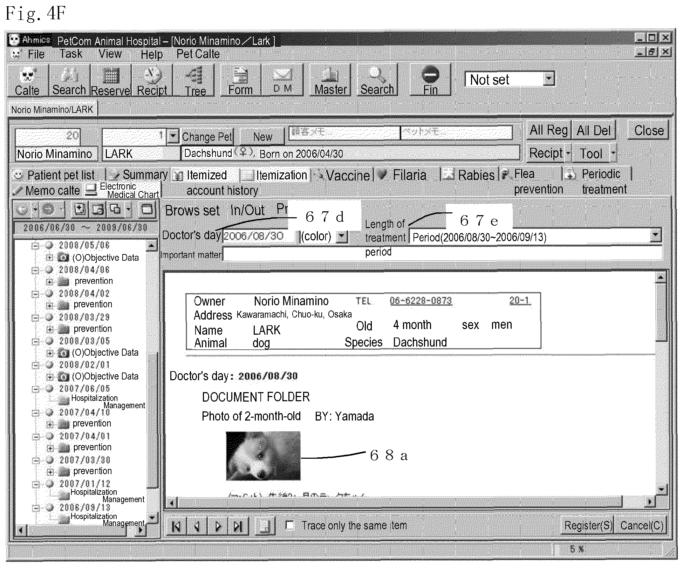

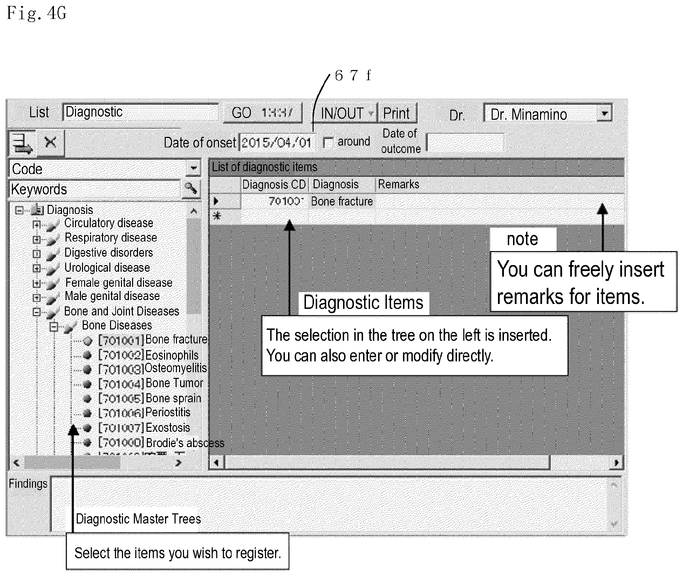

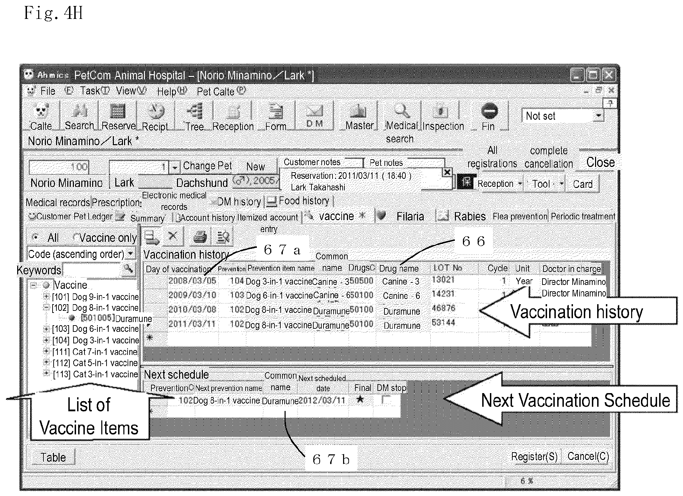

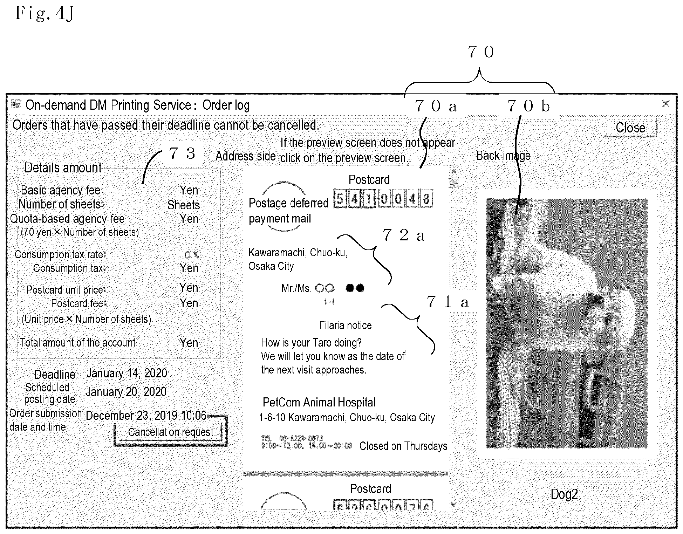

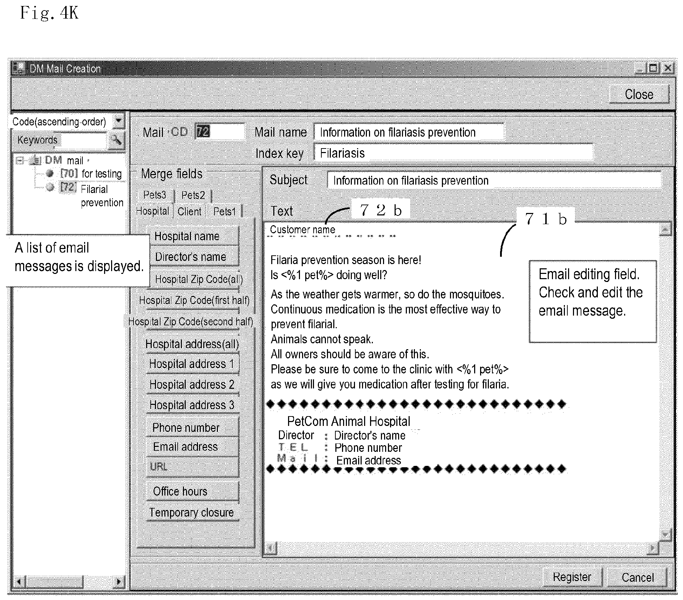

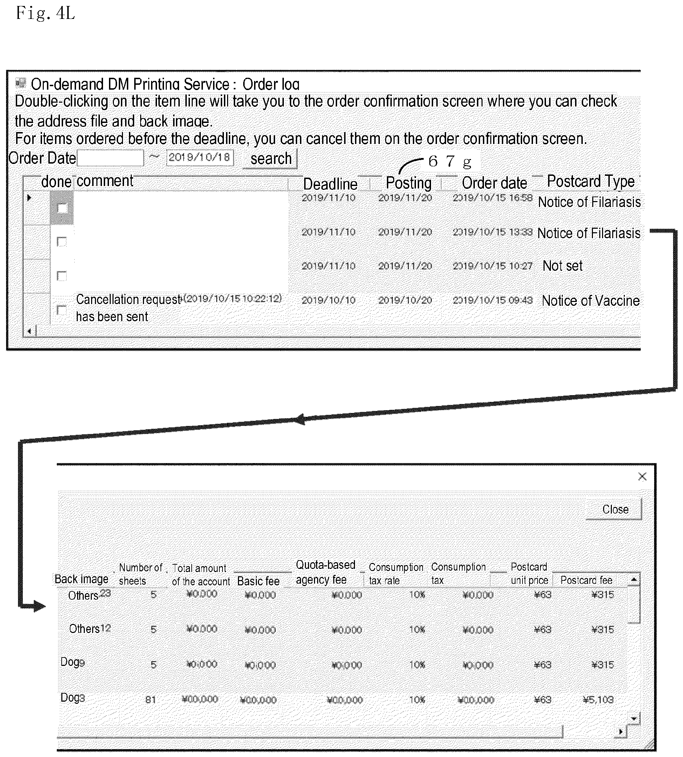

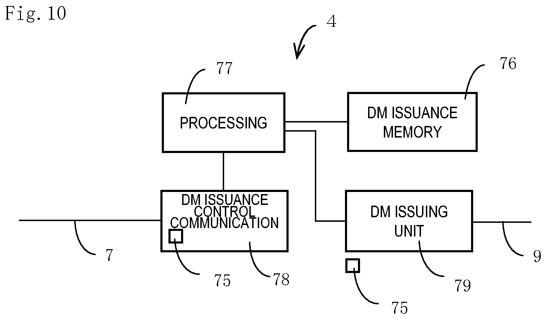

The present invention relates to a medical examination device for animals that can commission a task of issuing direct mail, the medical examination device characterized in that:
(a) a control processing device 1 installed at a veterinary hospital A and a DM issuing device 4 installed at a commission destination F commissioned with a task of issuing direct mail DM 75 are connected to each other via communication lines 5, 7;
(b) the control processing device 1 includes
   (b1) a first memory 10 that stores
      names and addresses 72a (Fig. 4J), 72b (Fig. 4K) that are delivery destinations corresponding to a plurality of animal owners,
      vaccination histories 67a, 67b (Fig. 4H) including a date for each animal and medical data histories 51 (Fig. 4A), 67c (Fig. 4E), 67d, 67e (Fig. 4G), 67f (Fig. 4H) including a date for each animal, and
      DM issuance information 49 including a predetermined text 71a (Fig. 4J) to prompt the owners to allow vaccination and a predetermined text 71b (Fig. 4K) for medical inquiry about the animals,
   (b2) a first processing circuit 17 that calculates an expected DM issuance date 67g (Fig. 4L) on the basis of the date of vaccination 67a, 67b (Fig. 4H) or the date of medical data 51 (Fig. 4A), 67c (Fig. 4E), 67d, 67e (Fig. 4F), 67f (Fig. 4G), creates the DM issuance information 49 (Fig. 3, Table 1) including the expected DM issuance date and the texts 71a (Fig. 4J), 71b (Fig. 4K) for vaccination or medical inquiry corresponding to the expected DM issuance date, and stores the DM issuance information 49 in the first memory 10, and
   (b3) a first control communication circuit 18 that transmits the DM issuance information 49 in the first memory 10 to the DM issuing device 4 via the communication lines 5, 7; and
(c) the DM issuing device 4 (Fig. 10) includes
   (c1) a DM issuance control communication circuit 78 that receives the DM issuance information 49 from the first control communication circuit 18,
   (c2) a DM issuance memory 76 that stores the DM issuance information 49 that has been received by the DM issuance control communication circuit 78, and
   (C3) a DM issuing unit 79 that issues the DM to the delivery destination corresponding to the owner for each animal on the expected DM issuance date 67g (Fig. 4L) according to the DM issuance information 49 stored in the DM issuance memory 76.

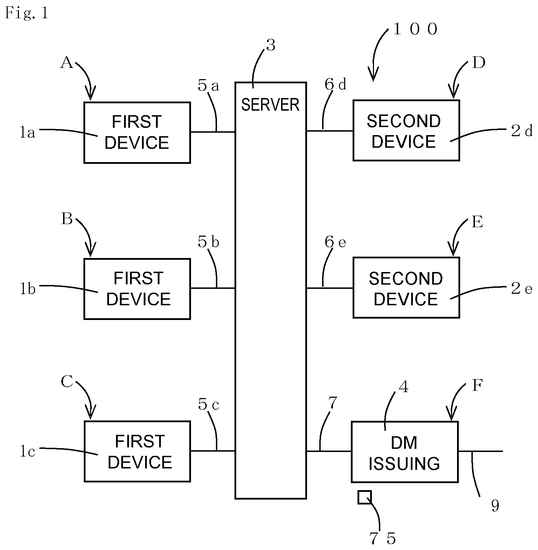

In an embodiment of the present invention, the DM issuing unit 79 prints on a postcard 70 or transmits an e-mail (Fig. 4K) to the address of each owner 72a (Fig. 4J), 72b (Fig. 4K) via the communication line 9 (Figs. 1 and 10).

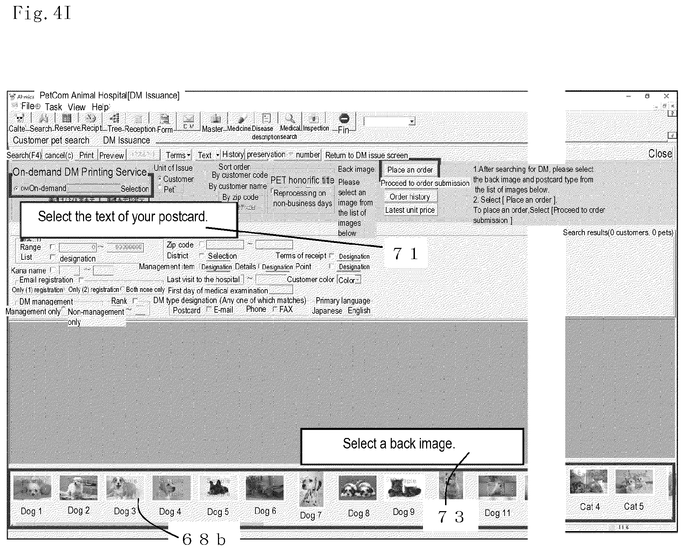

In an embodiment of the present invention, the DM issuance information 49 includes one of a plurality of predetermined photographs 68a (Fig. 4F), 68b (Fig. 4I) of each animal of identical kinds.

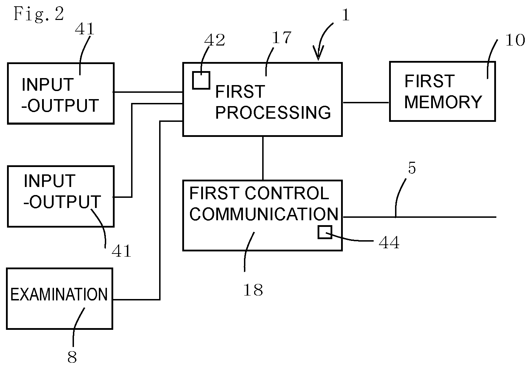

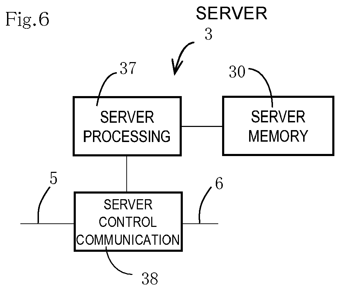

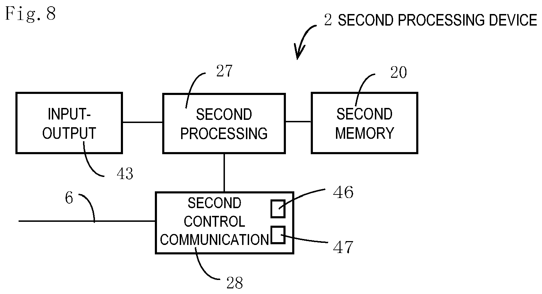

The present invention relates to a medical examination device for animals that can commission a task of issuing direct mail, the medical examination device including:
(a) a first control processing device 1 for a first physician installed at a first veterinary hospital A;
(b) a second control processing device 2 for a second physician installed at a second veterinary hospital D;
(c) a DM issuing device 4 installed at a commission destination F commissioned with the task of issuing direct mail DM 75; and
(d) a server 3 connected to the first control processing device 1, the second control processing device 2, and the DM issuing device 4 via communication lines 5, 6, 7 wherein:
(e) the first control processing device 1 (Fig. 2) includes:
   (e1) a first memory 10 that stores
      a first medical chart 11 (Fig. 3) provided for each of one or more animals to be given medical care, the first medical chart 11 including owner information about an owner of each animal, identification information about each animal, medical data by the first physician, and examination data including an image,
      a second medical chart 12 created from the first medical chart 11 with at least part of the owner information excluded, and
      a master application program 14 for creating and outputting the first medical chart 11 and the second medical chart 12, and
      that further stores DM issuance information 49, the DM issuance information 49 including
         names and addresses 72a (Fig. 4J), 72b (Fig. 4K) that are delivery destinations corresponding to a plurality of the animal owners,
         vaccination histories 67a, 67b (Figs. 4H and 4I) including a date for each animal and medical data histories 51 (Fig. 4A), 67c (Fig. 4E), 67d, 67e (Fig. 4F), 67f (Fig. 4G) including a date for each animal, and
         a predetermined text 71a (Fig. 4J) to prompt the owners to allow vaccination and the predetermined text 71b (Fig. 4K) for medical inquiry about the animals,
      the first memory 10 further storing a medical diagnostic report 15 (Fig. 3) from the second control processing device 2 via the server 3 and the vaccination histories 67a, 67b (Fig. 4H) and the medical data histories 51 (Fig. 4A), 67c (Fig. 4E), 67d, 67e (Fig. 4F), 67f (Fig. 4G) including a date based on the medical diagnostic report 15 after being changed;
   (e2) a first processing circuit 17 that executes the master application program 14 to create the first medical chart 11 and the second medical chart 12 and to store them in the first memory 10; and
   (e3) a first control communication circuit 18 that
      outputs, to the server 3, a request signal 44 (Fig. 2) about the animal for which medical diagnosis is requested to the second physician in the second veterinary hospital D selected by the first physician, the second medical chart 12 of the animal for which the medical diagnosis is requested, the master application program 14, and further the DM issuance information 49,
      receives from the server 3 an acceptance signal 46 (Fig. 8) and the medical diagnostic report 15 (Fig. 3) by the second physician about the animal for which the medical diagnosis is requested and stores them in the first memory 10;
(f) the server 3 (Fig. 6) includes
   (f1) a server control communication circuit 38 that is connected to the first control communication circuit 18 and the second control processing device 2 via the communication lines 5, 6,
   (f2) a server memory 30, and
   (f3) a server processing circuit 37 that
      stores, in the server memory 30, the request signal 44, the second medical chart 12, and the master application program 14 from the first control communication circuit 18, which have been received by the server control communication circuit 38, transmits them to the second control processing device 2 by the server control communication circuit 38,
      stores, in the server memory 30, the DM issuance information 49 from the first control communication circuit 18, which has been received by the server control communication circuit 38, transmits the DM issuance information 49 to the DM issuing device 4 by the server control communication circuit 38,
      stores, in the server memory 30, the acceptance signal 46 (Fig. 8) and the medical diagnostic report 15 from the second control processing device 2, which have been received by the server control communication circuit 38, and transmits them to the first control communication circuit 18 by the server control communication circuit 38; and
(g) the second control processing device 2 (Fig. 8) includes
   (g1) a second control communication circuit 28 that receives the request signal 44 from the server control communication circuit 38 and, when accepting the medical care request for each animal, transmits the acceptance signal 46 for each animal to the server control communication circuit 38,
   (g2) a second memory 20 that stores the second medical chart 12 and the master application program 14 downloaded from the server 3 for each animal corresponding to the acceptance signal 46, and
   (g3) a second processing circuit 27 that executes the master application program 14 stored in the second memory 20 to create the medical diagnostic report 15 based on the second medical chart 12 by the second physician,
   (g4) the second control communication circuit 28 transmitting the medical diagnostic report 15 from the second processing circuit 27 to the server 3; and
(h) the DM issuing device 4 (Fig. 10) includes
   (h1) a DM issuance control communication circuit 78 that receives the DM issuance information 49 from the first control communication circuit 18 via the server control communication circuit 38,
   (h2) a DM issuance memory 76 that stores the DM issuance information 49 that has been received by the DM issuance control communication circuit 78, and
   (h3) a DM issuing unit 79 that issues the DM to the delivery destination corresponding to the owner for each animal on the expected DM issuance date 67g (Fig. 4L) according to the DM issuance information 49 stored in the DM issuance memory 76.

The present invention relates to a medical examination device for animals that can commission a task of issuing direct mail, the medical examination device including:
(a) a first control processing device 1 for a first physician installed at a first veterinary hospital A;
(b) a second control processing device 2 for a second physician installed at a second veterinary hospital D;
(c) a DM issuing device 4 installed at a commission destination F commissioned with the task of issuing direct mail DM 75; and
(d) a server 3 connected to the first control processing device 1, the second control processing device 2, and the DM issuing device 4 via communication lines 5, 6, 7, wherein:
(e) the first control processing device 1 (Fig. 2) includes
   (e1) a first memory 10 that stores
      a first medical chart 11 (Fig. 3) provided for each of one or more animals to be given medical care, the first medical chart 11 including owner information about an owner of each animal, identification information about each animal, medical data by the first physician, and examination data including an image,
      a second medical chart 12 created from the first medical chart 11 with at least part of the owner information excluded,
      additional examination data 16 added by the first physician in response to an additional request signal 47 (Fig. 8), and
      a master application program 14 for creating and outputting the first medical chart 11, the second medical chart 12, and the additional examination data 16, and
      that further stores DM issuance information 49, the DM issuance information 49 including
         names and addresses 72a (Fig. 4J), 72b (Fig. 4K) that are delivery destinations corresponding to a plurality of the animal owners,
         vaccination histories 67a ,67b (Fig. 4H) including a date for each animal and medical data histories 51 (Fig. 4A), 67c (Fig. 4E), 67d, 67e (Fig. 4F), 67f (Fig. 4G) including a date for each animal, and
         a predetermined text 71a (Fig. 4J) to prompt the owners to allow vaccination and a predetermined text 71b (Fig. 4K) for medical inquiry about the animals,
   the first memory 10 storing a medical diagnostic report 15 (Fig. 3) from the second control processing device 2 via the server 3 and vaccination histories 67a, 67b (Fig. 4H) and medical data histories 51 (Fig. 4A), 67c (Fig. 4E), 67d, 67e (Fig. 4F), 67f (Fig. 4G) including a date based on the medical diagnostic report 15 after being changed,
   (e2) a first processing circuit 17 that executes the master application program 14 to create the first medical chart 11, the second medical chart 12, and the additional examination data 16 (Fig. 3) and to store them in the first memory 10, and
   (e3) a first control communication circuit 18 that
      outputs, to the server 3, a request signal 44 (Fig. 2) about the animal for which medical diagnosis is requested to the second physician in the second veterinary hospital D selected by the first physician, the second medical chart 12 of the animal for which the medical diagnosis is requested, the master application program 14, the additional examination data 16 (Fig. 3) in response to the additional request signal 47 (Fig. 8), and further the DM issuance information 49,
      receives from the server 3 an acceptance signal 46 (Fig. 8) and the medical diagnostic report 15 (Fig. 3) by the second physician about the animal for which the medical diagnosis is requested and stores them in the first memory 10;
(f) the server 3 (Fig. 6) includes
   (f1) a server control communication circuit 38 that is connected to the first control communication circuit 18 and the second control processing device 2 via the communication lines 5, 6,
   (f2) a server memory 30, and
   (f3) a server processing circuit 37 that
      stores, in the server memory 30, the request signal 44, the second medical chart 12, the additional examination data 16, and the master application program 14 from the first control communication circuit 18, which have been received by the server control communication circuit 38, transmits them to the second control processing device 2 by the server control communication circuit 38, and
      further stores, in the server memory 30, the DM issuance information 49 from the first control communication circuit 18, which has been received by the server control communication circuit 38, transmits the DM issuance information 49 to the DM issuing device 4 by the server control communication circuit 38,
      stores, in the server memory 30, the acceptance signal 46 (Fig. 8), the additional request signal 47, and the medical diagnostic report 15 from the second control processing device 2, which have been received by the server control communication circuit 38, and transmits them to the first control communication circuit 18 by the server control communication circuit 38; and
(g) the second control processing device 2 (Fig. 8) includes
   (g1) a second control communication circuit 28 that receives the request signal 44 from the server control communication circuit 38 and, when accepting the medical care request for each animal, transmits the acceptance signal 46 for each animal to the server control communication circuit 38,
   (g2) a second memory 20 that stores the second medical chart 12, the additional examination data 16, and the master application program 14 downloaded from the server 3 for each animal corresponding to the acceptance signal 46, and
   (g3) a second processing circuit 27 that executes the master application program 14 stored in the second memory 20 to output the additional request signal 47 (Fig. 8) for requesting examination data not included in the second medical chart 12 as the additional examination data 16 (Fig. 3) and to create the medical diagnostic report 15 based on the additional examination data 16 by the second physician;
   (g4) the second control communication circuit 28 transmits the additional request signal 47 and the medical diagnostic report 15 from the second processing circuit 27 to the server 3; and
(h) the DM issuing device 4 (Fig. 10) includes
   (h1) a DM issuance control communication circuit 78 that receives the DM issuance information 49 from the first control communication circuit 18 via the server control communication circuit 38,
   (h2) a DM issuance memory 76 that stores the DM issuance information 49 that has been received by the DM issuance control communication circuit 78, and
   (h3) a DM issuing unit 79 that issues the DM to the delivery destination corresponding to the owner for each animal on the expected DM issuance date 67g (Fig. 4L) according to the DM issuance information 49 stored in the DM issuance memory 76. The second physician can create the medical diagnostic report 15 based on the additional examination data 16, and also can create the medical diagnostic report 15 based on the third medical chart 13 including the second medical chart 12 and the additional examination data 16, the third medical chart 13 being transmitted from the first control processing circuit 1 to the second control processing circuit 2 via the server 3. The present invention also includes such a configuration.

The present invention can implement the remote medical examination device 100 for animals that allows smooth, rapid, and advanced medical care for animals, and the remote medical examination method using the same. For example, a first physician who is a general physician in one or more of first veterinary hospitals A , B, and C (hereinafter, collectively referred to only as A) transmits a second medical chart 12 of an animal for which medical diagnosis is requested to at least one second physician who is a medical specialist in one or more of second veterinary hospitals D and E (hereinafter, collectively referred to only as D). The second physician performs medical diagnosis on the basis of the second medical chart 12 and creates an advanced medical diagnostic report 15 that is data indicating the result of the medical diagnosis in the form of text, graphics, photographs, and the like, and the first physician can perform medical diagnosis and medical treatment upon obtaining the medical diagnostic report 15. Moreover, the second physician can additionally request the first physician to send additional examination data 16 when the second medical chart 12 alone is not sufficient to diagnose the animal and create the medical diagnostic report 15. This allows the second physician to create more advanced medical diagnostic report 15 on the basis of data including the additional examination data 16 together with the second medical chart 12 and to provide it to the first physician.

In one embodiment of the present invention, the first control processing device 1 may transmit only the additional examination data 16 that is additionally requested by the second physician, or may create and transmit the third medical chart including the additional examination data 16 together with the second medical chart 12.

Since the first control processing device 1 in the first veterinary hospital A and the second control processing device 2 in the second veterinary hospital D are connected via the communication lines 5 and 6 to the server 3, advanced medical care of the animal can be performed smoothly and quickly.

From the first control processing device 1 of the first veterinary hospital A to the second control processing device 2 of the second veterinary hospital D, not only the second medical chart 12 and the additional examination data 16 in response to an additional request, or the third medical chart 13, but also the master application program 14 is transmitted at the same time. Therefore, even when the master application program 14 executed in a certain first control processing device 1 is different from the one already executed in the other second control processing device 2, the second medical chart 12 can be read in the second control processing device 2 by executing a new master application program 14 identical to that executed in the first control processing device 1. Accordingly, it is possible to read the additional examination data 16 in response to the additional request or the third medical chart 13, and to create the medical diagnostic report 15. Therefore, the medical diagnostic report 15 can be received and read in the first control processing device 1. The master application program 14 to be transmitted to the second veterinary hospitals D and E may only be a program for computational processing necessary for the second physician to create the medical diagnostic report 15 and necessary to additionally request the additional examination data 16 to the first physician. However, the master application program 14 may further include other programs, such as computational processing programs for all the execution environments of the respective first veterinary hospitals A, B, C.

As a result, it is possible to implement, for example, a configuration in which an unspecified number of first control processing devices 1 and an unspecified number of second control processing devices 2 in countries different from each other are connected to a common server 3. In this way, any one of a large number of physicians can receive the medical diagnostic report 15 from one or more of many second physicians, ensuring that advanced medical care based on the medical diagnostic report 15 can be provided.

In one embodiment of the present invention, when transmitting the second medical chart 12 to the second control processing device 2, the first control processing device 1 also transmits the master application program 14. Accordingly, when transmitting to the second control processing device 2 the additional examination data 16 or the third medical chart afterward, the first control processing device 1 may also transmit the master application program 14 though it is not necessarily needed to transmit the master application program 14.

The second control processing device 2 of the second physician is given the second medical chart 12 from the first control processing device 1 of the first physician, and the second medical chart 12 is the information excluding at least part of customer information (for example, names, addresses, and other information that can be used to identify specific owners) from the first medical chart 11. This makes it easy to obtain an objective diagnostic evaluation from the second physician. The first medical chart 11 includes a lot of customer information and pet information, which are used for medical care or search for various information for medical care in the first veterinary hospital A by the first processing circuit 17.

The first medical chart 11 may preferably include the dates of medical care and examination so as to be used for medical care through observation of the progress of medical care and examination results, but may not include the dates.

In this specification, the name and address 72a (Fig. 4J), 72b (Fig. 4K) of the owner in the customer information include an e-mail address of electric mails over the Internet, and the e-mail address is expressed in the form of "account name @(at mark) domain name", for example.

According to the present invention, the DM issuance information 49, stored in the first memory 10 of the control processing device 1 installed at the veterinary hospital A, is transmitted via the communication lines 5 and 7 and further via the server 3 and stored in the DM issuance memory 76 of the DM issuing device 4 installed at the commission destination F that is commissioned with the task of issuing direct mail 75, and the DM issuing unit 79 issues the DM 75 to the delivery destination 72a (Fig. 4J), 72b (Fig. 4K) corresponding to the owner for each animal according to the DM issuance information 49. The DM 75 is typically a postcard 70 and its concept also includes e-mail or the like. The DM issuance information 49 includes the vaccination histories 67a, 67b (Fig. 4H) including a date for each animal and medical data histories 51 (Fig. 4A), 67c (Fig. 4E), 67d, 67e (Fig. 4F), 67f (Fig. 4G) including a date for each animal. Furthermore, the vaccination histories 67a, 67b (Fig. 4H) and medical data histories 51 (Fig. 4A), 67c (Fig. 4E), 67d, 67e (Fig. 4F), 67f (Fig. 4G) including a date based on the medical diagnostic report 15 from the second control processing device 2 via the server 3 are changed, and as a result, the first processing circuit 17 calculates the expected DM issuance date 67g (Fig. 4L). Therefore, the DM 75, which contains the predetermined text 71a (Fig. 4J) to prompt vaccination for each animal to be given medical care in the veterinary hospital A and the predetermined text 71b (Fig. 4K) for medical inquiry about each animal, is issued on the expected DM issuance date 67g (Fig. 4L). In addition, the predetermined text 71a (Fig. 4J) to prompt the owner to allow vaccination and the predetermined text 71b (Fig. 4K) for medical inquiry about each animal in the DM issuance information 49 may be changed on the basis of the medical diagnostic report 15 by the first processing circuit 17. This makes it possible to greatly reduce the operation for issuing and sending direct mail (DM) 75 to the owner of each animal in the veterinary hospitals as much as possible.

The present invention can also implement the remote medical examination device 100 for animals that allows smooth, rapid, and advanced medical care for animals and a remote medical examination method using the same. For example, a first physician who is a general physician in the first veterinary hospital A can transmit the second medical chart 12 of an animal for which medical diagnosis is requested to a second physician who is a medical specialist in the second veterinary hospital D, the second physician performs medical diagnosis on the basis of the second medical chart 12 and creates an advanced medical diagnostic report 15 that is the data indicating the result of the medical diagnosis in the form of text, graphics, photographs, and the like, and the first physician can perform medical diagnosis and medical treatment upon obtaining the medical diagnostic report 15. Moreover, the second physician can additionally request the first physician to send additional examination data 16 when the second medical chart 12 alone is not sufficient to diagnose the animal and create the medical diagnostic report 15. This allows the second physician to create more advanced medical diagnostic report 15 on the basis of data including the additional examination data 16 along with the second medical chart 12 (e.g., the third medical chart 13) and to provide it to the first physician.

The second control processing device 2 of the second physician is given the second medical chart 12 from the first control processing device 1 of the first physician, and the second medical chart 12 is the information excluding at least part of customer information (for example, names, addresses, and other information that can be used to identify specific owners) from the first medical chart 11. The address of the owner of the animal, i.e., the name and the address 72a (Fig. 4J), 72b (Fig. 4K) of the delivery destination, is not transmitted to the second control processing device 2 of the second physician. This makes it easy to obtain an objective diagnostic evaluation from the second physician.

### Brief Description of Drawings

[Fig. 1] Fig. 1 is a block diagram illustrating the configuration of a remote medical examination device 100 for animals according to one embodiment of the present invention.
[Fig. 2] Fig. 2 is a block diagram illustrating the configuration of a first control processing device 1.
[Fig. 3] Fig. 3 is a block diagram partially showing the configuration of a first memory 10.

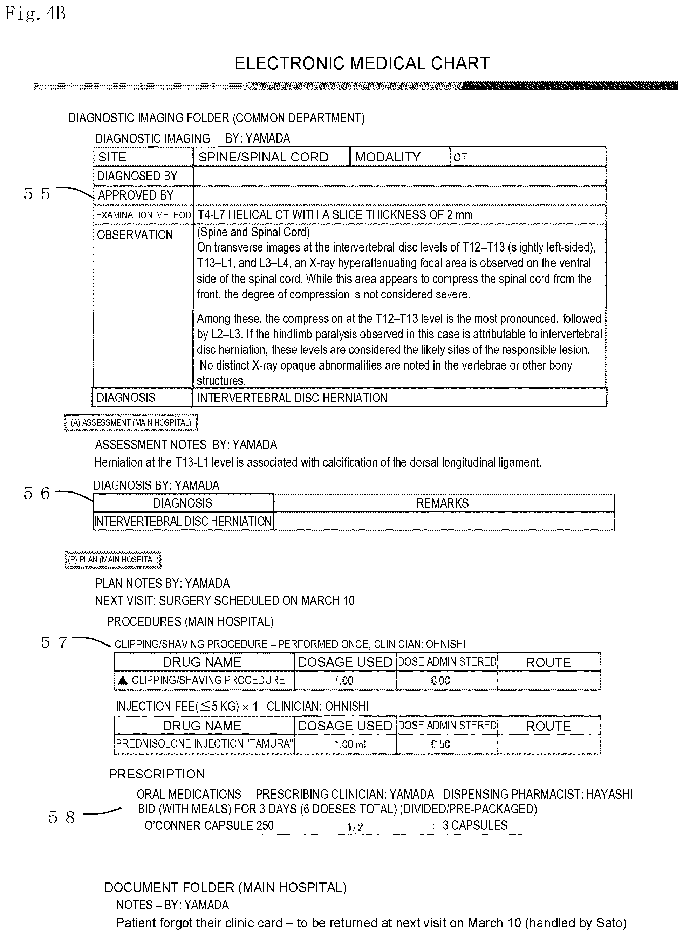

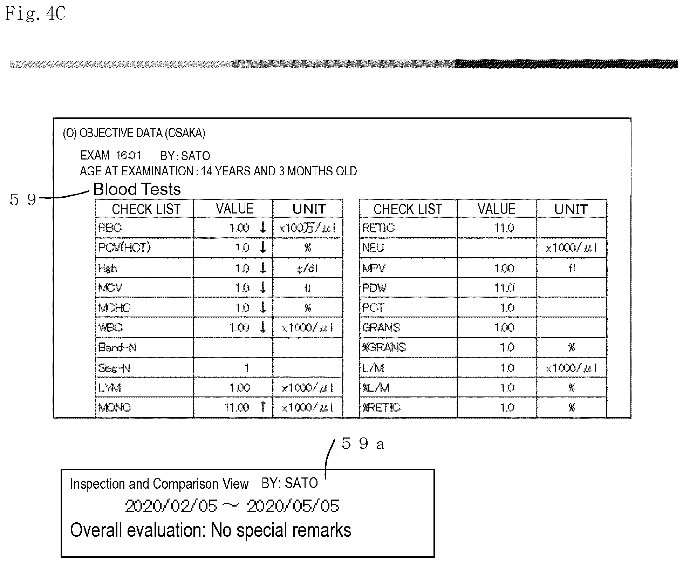

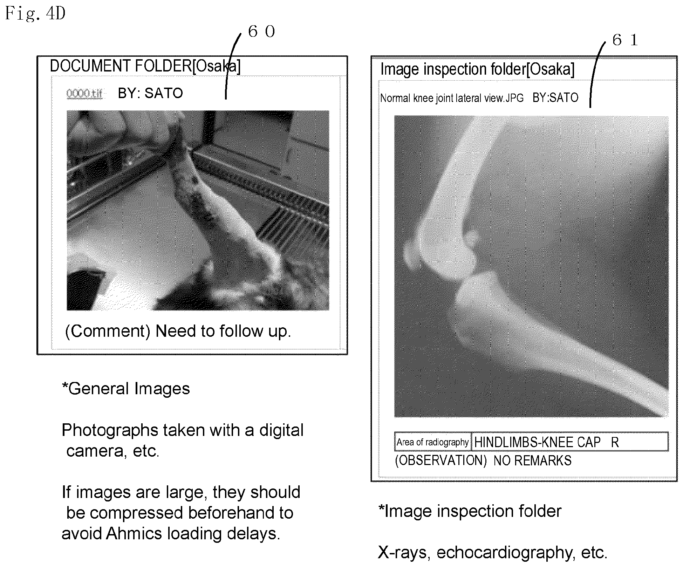

[Fig. 4] Figs. 4A to 4L are front views showing respective screens visually displayed on an input-output device 41 of the first control processing device 1.

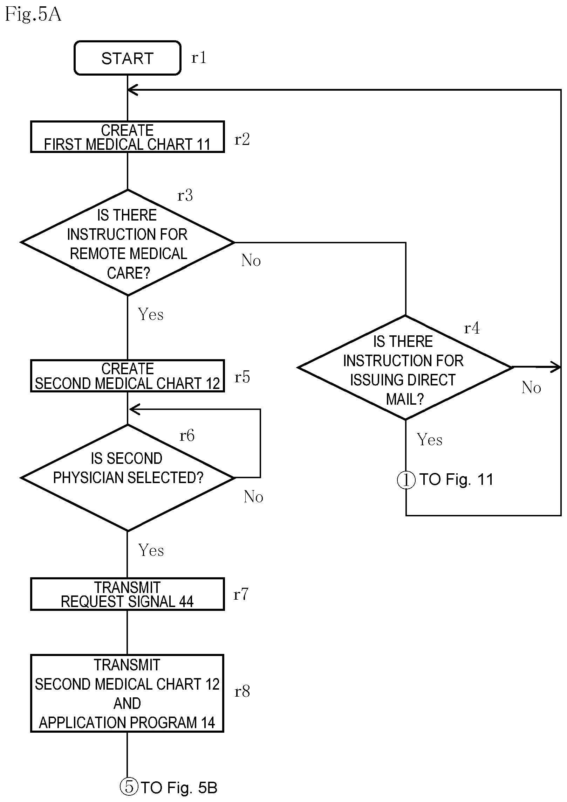

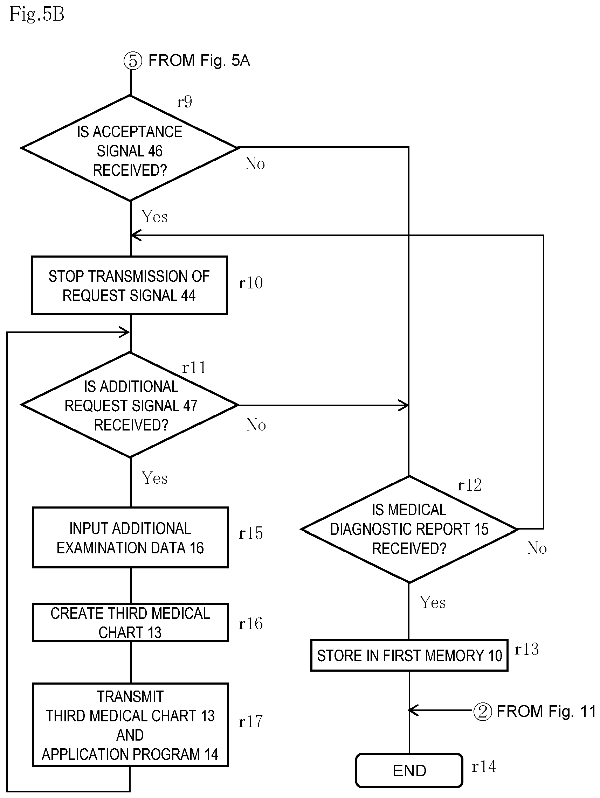

[Fig. 5] Figs. 5A and 5B are flowcharts for describing the operations of a first processing circuit 17 and a first control communication circuit 18.
[Fig. 6] Fig. 6 is a block diagram illustrating the configuration of a server 3.

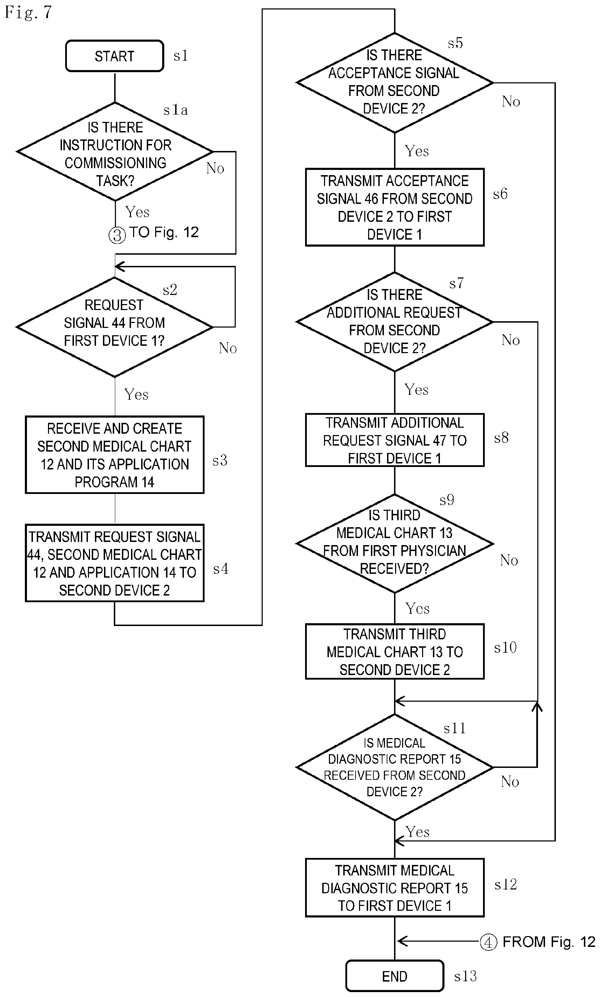

[Fig. 7] Fig. 7 is a flowchart for describing the operations of a server processing circuit 37 and a server control communication circuit 38.
[Fig. 8] Fig. 8 is a block diagram showing the configuration of a second control processing device 2.

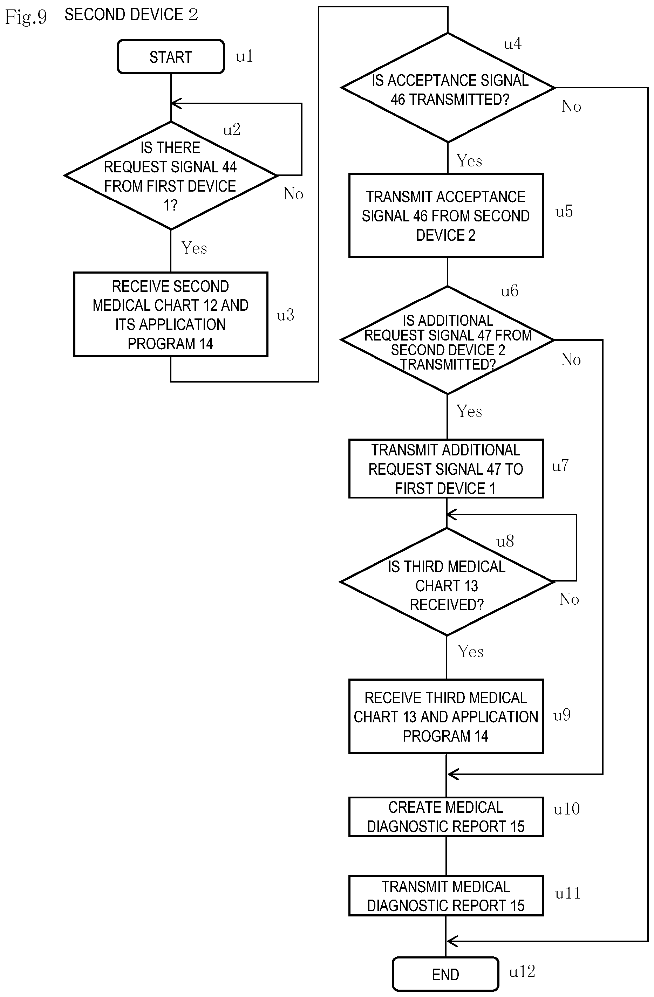

[Fig. 9] Fig. 9 is a flowchart for describing the operations of a second processing circuit 27 and a second control communication circuit 28.
[Fig. 10] Fig. 10 is a block diagram illustrating the configuration of a DM issuing device 4.

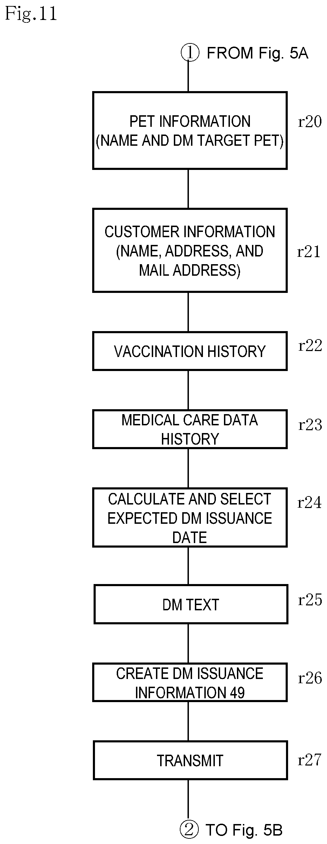

[Fig. 11] Fig. 11 is a flowchart consequent to Fig. 5 for describing the operations of the first processing circuit 17 and the first control communication circuit 18.

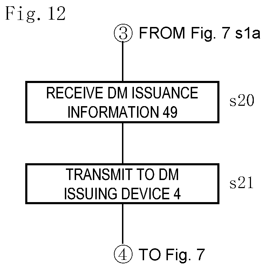

[Fig. 12] Fig. 12 is a flowchart consequent to Fig. 7 for describing the operations of the server processing circuit 37 and the server control communication circuit 38.

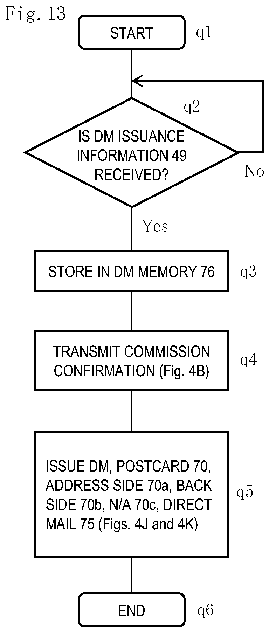

[Fig. 13] Fig. 13 is a flowchart for describing the operations of a DM issuance processing circuit 77, a DM issuance control communication circuit 78, and a DM issuing unit 79 of the DM issuing device 4.

### Description of Embodiments

In this specification, the animal owners may be referred to as customers from the viewpoint of the veterinary hospitals. A collection of information, data, and the like about the customers who are owners and about animals such as pets may be called medical charts, and each medical chart includes information and data resulting from computational processing for search, out of a large amount of information and data. For example, each medical chart may be implemented as a so-called electronic medical chart, which is used in systems that allow input/output and management of the contents, which are written on paper medical charts, by using personal computers. It should be understood that the spirit of the present invention is interpreted in a broader sense, not limited to customers and pets.

Fig. 1 is a block diagram illustrating the configuration of a remote medical examination device 100 for animals that can commission issuance of direct mail according to one embodiment of the present invention.

In recent years, veterinarians in veterinary hospitals have played important roles, and the information handled by veterinary hospitals has been increasing year by year. In the field of medical care for animals, veterinarians are so-called general physicians who supposedly provide general medical care for many kinds of animals, though it cannot be ignored that the veterinarians have their skilled and unskilled area depending on the medical fields. Therefore, it may not be possible to provide smooth, rapid and advanced medical care by general physicians alone. In order to solve this problem, some countries have established a medical specialist system for each medical specialty of veterinarians.

For accurate remote medical checkup of pets and other animals, a support system has been proposed which improves the quality of images of the bodies of the animals captured by owners by cameras and the quality of voice between the owners and the veterinarians (Japanese Patent Laid-Open Publication No. 2022-44667 described before). According to the conventional technology, for example, the medical care in a medical field where a certain general physician who is a veterinarian is not skilled cannot be supplemented by the medical knowledge of a medical specialist who is another veterinarian skilled in the medical field. Accordingly, there are needs for more advanced medical care for animals. According to the present invention, the remote medical examination device 100 that can commission issuance of direct mail allows smooth, rapid and advanced medical care for animals.

The remote medical examination device 100 that can commission issuance of direct mail allows smooth, rapid and advanced medical care for animals.

The remote medical examination device 100 for animals is configured by including: (a) first control processing devices 1a, 1b, 1c installed at a plurality of first veterinary hospitals A, B, C for first physicians such as general physicians; (b) second control processing devices 2d, 2e installed at a plurality of second veterinary hospitals D, E for second physicians such as medical specialists; and (c) a server 3 connected to each of the first control processing devices 1 and the second control processing devices 2 via communication lines 5a, 5b, 5c; 6d, 6e. The server 3 is further connected to a direct mail (abbreviated as DM) issuing device 4, which is installed at a commission destination F of the first veterinary hospital A via a communication line 7.

Fig. 2 is a block diagram illustrating the configuration of the first control processing device 1. The first control processing device 1 includes a first memory 10, a first processing circuit 17, and a first control communication circuit 18. The first processing circuit 17 is connected to a keyboard or the like for inputting medical data, and an input-output device 41 for output display, and is equipped with a clock circuit 42 that outputs signals representing the date and time of medical data and examination data, and current date and time such as input date and time. The first processing circuit 17 is given data such as examination data by an examination device 8. For example, as will be described later, the examination device 8 outputs blood-related examination data, photographic examination data showing pathological states by X-ray, ultrasound, and microscope images, and other examination data to the first processing circuit 17, and stores them in the first memory 10.

Fig. 3 is a block diagram partially illustrating the configuration of the first memory 10. The first memory 10 stores data including a first medical chart 11, a second medical chart 12, a third medical chart 13, a master application program 14, and a medical diagnostic report 15 by a second physician. The first medical chart 11, which is prepared for each of one or more animals to be given medical care, includes customer information about the customer who is the owner of each pet, pet information that is identification information about each pet that is animal, and medical care-related information 19 by the first physician, and further includes so-called header information, such as the name of the first veterinary hospital A, and the name of the second veterinary hospital D, if there is the second veterinary hospital D. The customer information includes customer's name, address, e-mail address, and customer code. The pet information includes pet name, whether the pet is a predetermined direct mail (abbreviated to DM) target or not, and pet code. The customer code and the pet code are identification codes which are constituted of, for example, numbers only so as to prevent the second physicians other than the first physician from identifying the specific customer and specific pet. The medical care-related information 19 includes later-described medical data 52, 53, 55 to 57 with respect to medical checkup, medical diagnosis, and medical treatment, examination date 54 including images, and data on date and history of vaccination, medical care, and examination.

The second medical chart 12 includes the customer code and the pet code, out of the customer information and the pet information in the first medical chart 11, and further includes the medical care-related information 19 in the first medical chart 11.

The third medical chart 13 includes additional examination data 16 which is added to the second medical chart 12 by the first physician in response to an additional request signal 47 (see Fig. 8) from the second control processing device 2 by the second physician as will be described later.

The first memory 10 further stores document information 48 and DM issuance information 49. The document information 48 includes customer payment with respect to accounting, pet medication (prescription, dispensing) with respect to medicine, food and diet, and hair trimming.

The DM issuance information 49 is information supplied from the first veterinary hospital A to the commission destination F such as companies commissioned with the operation for issuing and sending DM to each customer. As illustrated in Fig. 3, examples of main contents of the DM issuance information 49 include the e-mail address of the commission destination F, texts, summer greetings, new year's greetings, and photographs of pets, and further include the name, address, e-mail address, and the like of each customer required to send the DM. The text is the content to be placed in the e-mails, postcards, summer greeting cards, new year greeting cards, or the like, to be sent to the customers who are the predetermined DM targets. Examples of the text include notifications for vaccination, prevention of filaria, fleas, and the like for pets, and filaria.

Examples of more detailed contents of the DM issuance information 49 are as shown in Table 1 below. The DM issuance information 49 is divided into three categories: I, II and III, i.e., I for each vaccination, II for each customer, and III for each pet. The DM issuance information 49 includes at least: the names and the addresses 72a (Fig. 4J), 72b (Fig. 4K) that are delivery destinations corresponding to a plurality of animal owners; vaccination histories 67a, 67b (Fig. 4H) including a date for each animal and medical data histories 51 (Fig. 4A), 67c (Fig. 4E), 67d, 67e (Fig. 4F), 67f (Fig. 4G) including a date for each animal; and a predetermined text 71a (Fig. 4J) to prompt the owners to allow vaccination and a predetermined text 71b (Fig. 4K) for medical inquiry about the animals.

The first processing circuit 17 further appropriately change and correct the vaccination histories 67a, 67b (Fig. 4H) and medical data histories 51 (Fig. 4A), 67c (Fig. 4E), 67d, 67e (Fig. 4F), 67f (Fig. 4G) including a date on the basis of the medical diagnostic report 15 from the second control processing device 2 via the server 3. As a result, the first processing circuit 17 calculates, for example, the day when a predetermined number of days elapses from the last vaccination date to obtain an expected DM issuance date 67g (Fig. 4L), and further appropriately changes and corrects the predetermined text 71a (Fig. 4J) to prompt the owners to allow vaccination and the predetermined text 71b (Fig. 4K) for medical inquiry about the animals in the DM issuance information 49. Therefore, the DM containing the predetermined text 71a (Fig. 4J) to prompt owners to allow vaccination for each animal that is medically consulted in the veterinary hospital A and the predetermined text 71b (Fig. 4K) for medical inquiry about each animal is issued on the expected DM issuance date 67g (Fig. 4L). In addition, the predetermined text 71a (Fig. 4J) to prompt the owner to allow vaccination and the predetermined text 71b (Fig. 4K) for medical inquiry about each animal in the DM issuance information 49 may be changed on the basis of the medical diagnostic report 15 by the first processing circuit 17. In this way, the contents of the text of the DM may include notification for vaccination, notification for filaria, medical care inquiries, follow-up after recuperation or surgery, medication (prescription, preparation), food and diet, hair trimming, and others. This makes it possible to greatly reduce the operations for issuing and sending direct mail (DM) to the owner of each animal in the veterinary hospital A as much as possible.

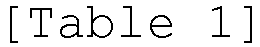

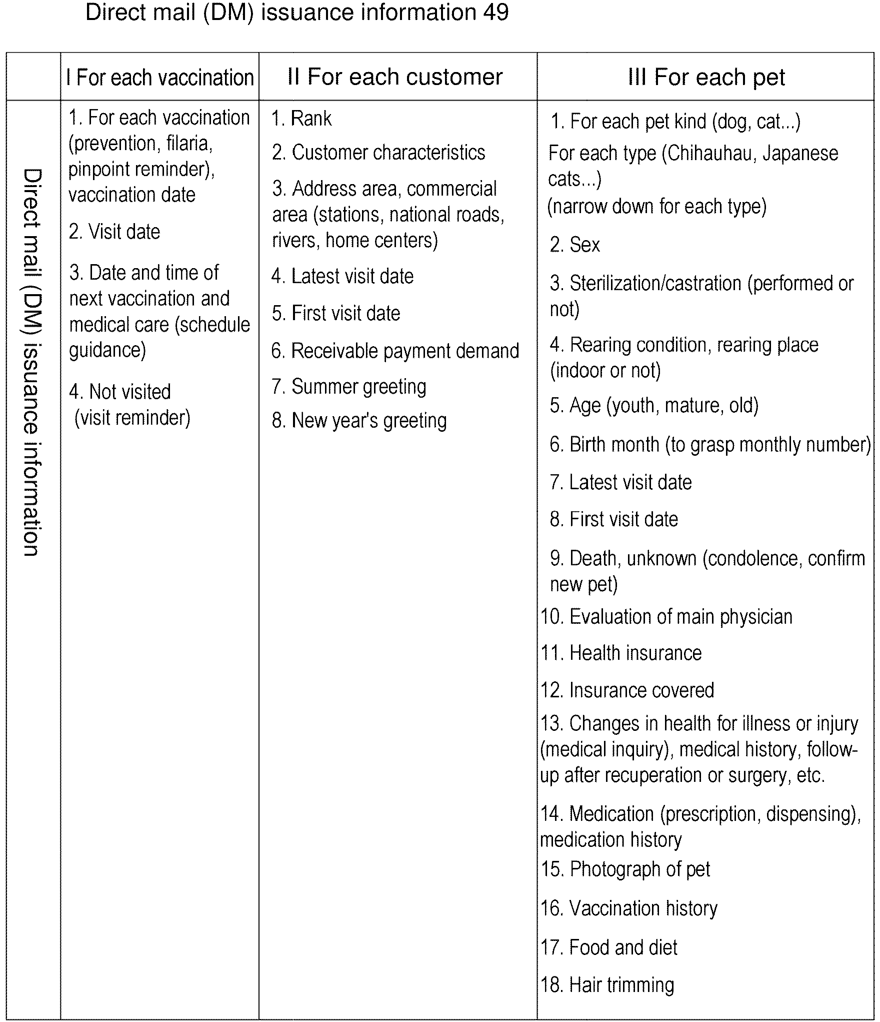

Fig. 4 includes front views showing respective screens visually displayed on the input-output device 41 of the first control processing device 1. The content for each pet displayed on each screen is stored in the first memory 10. Of these screens, Figs. 4A to 4H show specific examples of some of the medical care-related information 19 contained in the first to third medical charts 11 to 13, Figs. 4J to 4K show specific examples of the DM issuance information 49, and Fig. 4L shows the status of the DM issuance information 49 transmitted to the DM issuing device 4. The data in Fig. 4L partially include information that are transmitted from the DM issuing device 4 and received by the first control processing device 1. In these drawings, the names of customers, pets, and the like are pseudonyms for the convenience of understanding.

Fig. 4A includes medical care and examination date 51, medical data 52, 53, and examination data 54 made of images such as X-ray photographs. ultrasound images, and the like images. Fig. 4B includes medical data 55 to 57, medicine 58 to be used, and the like data. Fig. 4C includes blood test data 59 of the pet, its date 59a, and the like data. Fig. 4D includes examination data made of images of a pet such as a body appearance photograph 60, an X-ray photograph 61, and the like images and may further include a photograph, which is ultrasound examination data. Fig. 4E includes data 62 on blood chemical cleaning test of a pet, a body appearance photograph 63, a photograph 64 that is microscopic data of pathological examination, and the like images, and also includes medical data 65, which is a medical treatment policy, their dates 67c, and the like data. Fig. 4F includes medical data for a pet, its date 67d, duration of medical treatment 67e, a photograph of the pet 68a, the kind (e.g., dog) and breed (for example, a dachshund) of the pet, the name of the pet, the name and the address of the owner, and the like data. Fig. 4G includes medical data for fracture or the like of the pet, its date 67f, and the like data. Fig. 4H includes a history 66 including a vaccination date 67a, a vaccination reservation 67b, and the like data. Fig. 4I includes, as the DM issuance information 49 for issuing DM, a photograph 68b of the pet that is selected to be placed on a back side 70b of a postcard 70 in Fig. 4J according to the content 73 to be placed for the convenience of explanation. Fig. 4I also allows selection of the predetermined text 71a (Fig. 4J) to prompt the owner to allow vaccination and the predetermined text 71b (Fig. 4K) for medical inquiry about the animal according to the content 71 to be placed for the convenience of explanation. In Fig. 4J, the text 71a to notify filaria to prompt owner to allow vaccination and the name and address 72a of the owner that is the delivery destination are placed on an address side 70a of the DM postcard 70, and the photograph of the pet of the owner or the photograph of other pets is placed on the back side 70b. In addition, data 73 about an expense of DM issuance by the commission destination F, the number of postcards 70, or the like are included. In Fig. 4K, a mail address 72b of the owner that is the delivery destination is placed, in addition to the notification of filaria in the text 71b prepared for electric DM and the predetermined text 71b for medical inquiry about the pet. In Fig. 4L, a scheduled drop date 67g of the postcard 70, which is the expected DM issuance date by the commission destination F commissioned with the task of issuing the direct mail 70, its cost, and the number of the postcards are placed.

With reference to Figs. 2 and 3 again, the first processing circuit 17 executes the master application program 14 stored in the first memory 10 to create the first to third medical charts 11 to 13 and store them in the first memory 10. The first control communication circuit 18 outputs, to the server 3, a request signal 44 about the animal for which medical diagnosis is requested to the second physician in the second veterinary hospital D selected by the first physician, the second medical chart 12 of the pet for which the medical diagnosis is requested, the master application program 14, and the third medical chart 13 corresponding to the additional request signal 47 (Fig. 8), receives from the server 3 an acceptance signal 46 (Fig. 8) and the medical diagnostic report 15 (Fig. 3) by the second physician about the pet for which the medical diagnosis is requested and stores them in the first memory 10. The first processing circuit 17 and the first control communication circuit 18 are implemented by a computer that executes the master application program 14 or other programs.

Fig. 5 is a flowchart for describing operations of the first processing circuit 17 and the first control communication circuit 18. The process proceeds from step r1 to step r2, and the first medical chart 11 of a pet to be given medical care is created. When there is no instruction for remote medical care by the first physician through the input-output device 41 in step r3, it is determined whether there is an instruction to commission the task of issuing DM in step r4. When it is determined that there is an instruction for remote medical care in step r3, the second medical chart 12 is created in step r5. When the first physician specifies one or more second physicians in the second veterinary hospital or hospitals D as a request destination in step r6, the first control communication circuit 18 outputs to the server 3 a request signal 44 in step r7, and outputs the second medical chart 12 and the master application program 14 in step r8 for the specified second veterinary hospital D.

The second control processing device 2 in the specified second veterinary hospital D receives the request signal 44 via the server 3, and executes the master application program 14, so that the second physician examines the second medical chart 12. As a result, when the second physician accepts the request for medical care, an acceptance signal 46 is transmitted from the second control processing device 2 (see step u5 in Fig. 9 described later). The acceptance signal 46 is given to the first control communication circuit 18 via the server 3 and is received in step r9, by which the output of the request signal 44 is stopped in step r10. In another embodiment of the present invention, when it is desired to obtain the medical diagnostic reports 15 on the basis of respective acceptances from each of a plurality of second veterinary hospitals D that are request destination for medical care, the output of the request signal 44 may be stopped at the point of time when the counted number of acceptance signals 46 from the respective second veterinary hospitals D reaches the number of requests for medical care.

In step r11, it is determined whether or not the additional request signal 47 has been received from the second control processing device 2 of the second veterinary hospital D via the server 3. When the second physician determines that the second medical chart 12 alone is not sufficient for accurate medical diagnosis of the pet, and thus, requests the first physician to additionally send the additional examination data 16 in the second control processing device 2, the additional request signal 47 for the additional examination data 16 is transmitted, and is received in the first control communication circuit 18 via the server 3.

When the additional request signal 47 is not received in step r11, the medical diagnostic report 15 created only on the basis of the second medical chart 12 by the second physician is received from the second control processing device 2 in step r12, and is stored in the first memory 10 in step r13. The first physician can determine a medical treatment policy for the pet on the basis of the medical diagnostic report 15 by the second physician and can thereby provide advanced medical care. In next step r14, the operation is ended.

When the additional request signal 47 is received in step r11, the first physician creates and inputs the additional examination data 16 required by the additional request signal 47 in step r15, and the additional examination data 16 is stored in the first memory 10 by the first processing circuit 17 in step r16. The first processing circuit 17 creates the third medical chart 13 including the second medical chart 12 and also the additional examination data 16 for the second physician. In step r17, the first control communication circuit 18 transmits the third medical chart 13 and the master application program 14 to the second control processing device 2 via the server 3, receives the medical diagnostic report 15 created by the second physician based the third medical chart 13 including the second medical chart 12 and also the additional examination data 16 in step r12, and stores the medical diagnostic report 15 in the first memory 10 in step r13. Thus, the first physician can determine a medical treatment policy for the pet on the basis of the medical diagnostic report 15 created by the second physician on the basis of the third medical chart 13, and thereby provide advanced medical care.

In other embodiments of the present invention, in place of steps r16 and r17, transmission of the information of the second medical chart 12 and the master application program 14, which are previously transmitted in step r8, may be omitted, and only the additional examination data 16 may be transmitted, or the additional examination data 16 and a pet code or the like to identify the additional examination data 16 may also be transmitted without creation and transmission of the third medical chart 13.

The request signal 44 may include a code signal or the like that identifies the first control processing device 1 that has transmitted the request signal 44, and the acceptance signal 46 and the additional request signal 47 may include a code signal or the like that identifies the second control processing device 2 that has transmitted the acceptance signal 46 and the additional request signal 47, so that the first control processing device 1 and the second control processing device 2 can accurately exchange signals such as information and data.

Fig. 6 is a block diagram illustrating the configuration of the server 3. The server 3 includes the server control communication circuit 38, the server memory 30, and a server processing circuit 37. The server control communication circuit 38 is connected to the first control communication circuit 18 and the second control processing device 2 via the communication lines 5, 6.

The server processing circuit 37 stores, in the server memory 30, the request signal 44, the second medical chart 12, the third medical chart 13, and the master application program 14, from the first control communication circuit 18, which have been received by the server control communication circuit 38, and transmits them to the second control processing device 2 by the server control communication circuit 38. Furthermore, the server processing circuit 37 stores, in the server memory 30, the acceptance signal 46, the additional request signal 47, and the medical diagnostic report 15 from the second control processing device 2, which have been received by the server control communication circuit 38, and transmits them to the first control communication circuit 18 by the server control communication circuit 38. The server processing circuit 37 and the server control communication circuit 38 are implemented by a computer that executes the master application program 14 or the like programs.

Fig. 7 is a flowchart for describing operations of the server processing circuit 37 and the server control communication circuit 38. The process proceeds from step s1 to step s1a. Here, if the signal for commissioning the issuance of DM has not been received from the first control processing device 1, the process proceeds to next step s1b. When it is determined that the request signal 44 by executing step r7 of Fig. 5 described above from the first control communication circuit 18 has been received, the second medical chart 12 and the master application program 14 by executing step r8 of Fig. 5 described above from the first control communication circuit 18 is received in step s2. Then, the server processing circuit 37 stores them in the server memory 30. In next step s3, the request signal 44, the second medical chart 12, and the master application program 14 stored in the server memory 30 are transmitted from the server control communication circuit 38 to the second control processing device 2.

In step s4, it is determined whether the server control communication circuit 38 has received the acceptance signal 46 from the second control processing device 2. The second control processing device 2 of the second veterinary hospital D specified by the first physician receives the request signal 44 from the server 3 as described above, and executes the master application program 14. Furthermore, the second physician examines the second medical chart 12. As a result, when the second physician accepts the request for medical care, an acceptance signal 46 is transmitted from the second control processing device 2 to the server 3. If the server control communication circuit 38 does not receive the acceptance signal 46, the operation is ended in step s5.

If the server control communication circuit 38 receives the acceptance signal 46 from the second control processing device 2 in step s4, the server processing circuit 37 stores it in the server memory 30, and transmits the acceptance signal 46 to the first control processing device 1 in step s6, so that the step r9 of Fig. 5 as described above is executed.

In step s7, it is determined whether the server control communication circuit 38 has received the additional request signal 47 from the second control processing device 2. If the additional request signal 47 is not received in the step s7, upon receiving the medical diagnostic report 15, created only on the basis of the second medical chart 12 by the second physician, from the second control processing device 2 in the subsequent step s11, the server processing circuit 37 stores the medical diagnostic report 15 in the server memory 30 in step s12, and the server control communication circuit 38 transmits the medical diagnostic report 15 to the first control processing device 1. Thus, the first control processing device 1 executes step r12 of Fig. 5 described above.

The second physician determines that the second medical chart 12 alone is not sufficient for accurate medical diagnosis of the pet, and thus, requests the first physician to additionally send the additional examination data 16. Accordingly, the second control processing device 2 transmits the additional request signal 47 for the additional examination data 16.

When the server control communication circuit 38 receives the additional request signal 47 in step s7, the server processing circuit 37 stores it in the server memory 30. The server control communication circuit 38 transmits the additional request signal 47 to the first control processing device 1 in step s8, so that step r11 of Fig. 5 described above is executed. Accordingly, the first physician creates the additional examination data 16 requested by the additional request signal 47, and the third medical chart 13 for the second physician including the second medical chart 12 and also the additional examination data 16 is created and transmitted in steps r16 and r17 of Fig. 5 described above.

When the server control communication circuit 38 receives the third medical chart 13 from the first control communication circuit 18 in step s9, the server processing circuit 37 stores it in the server memory 30, and the server control communication circuit 38 transmits it to the second control processing device 2 in step s10. Thus, the second physician can create the medical diagnostic report 15 on the basis of the third medical chart 13 including the second medical chart 12 and also the additional examination data 16. When the server control communication circuit 38 receives the medical diagnostic report 15 based on the third medical chart 13 from the second control processing device 2 in the same manner as described above in step s11, the server processing circuit 37 stores it in the server memory 30, and the server control communication circuit 38 transmits the medical diagnostic report 15 to the first control processing device 1. Thus, the first physician can provide advanced medical care based on the medical diagnostic report 15 created by the second physician on the basis of the third medical chart 13.

Fig. 8 is a block diagram illustrating a configuration of the second control processing device 2. The second control processing device 2 includes the second control communication circuit 28, the second memory 20, and the second processing circuit 27. The second memory 20 stores the second medical chart 12, the third medical chart 13, and the master application program 14 downloaded from the server 3 for each pet corresponding to the acceptance signal 46. The second control communication circuit 28 communicates with the server control communication circuit 38 to receive the request signal 44. When the second physician accepts the request for medical care for each pet, the second control communication circuit 28 transmits an acceptance signal 46 for each pet, transmits the additional request signal 47, from the second processing circuit 27, for requested the examination data not included in the second clinical record 12 as the additional examination data 16 by the second physician, receives the additional examination data 16 by the first physician in response to the additional request signal 47, and transmits the medical diagnostic report 15 from the second processing circuit 27. The second processing circuit 27 executes the master application program 14 stored in the second memory 20 to output the additional request signal 47 for requesting the examination data not included in the second medical chart 12 as the additional examination data 16, and creates the medical diagnostic report 15 based on the third medical chart 13 by the second physician.

Fig. 9 is a flowchart for describing operations of the second processing circuit 27 and the second control communication circuit 28. The process proceeds from step u1 to step u2. When it is determined that the request signal 44 by executing step r7 in Fig. 5 described above has been received from the first control communication circuit 18 via the server 3, the second medical chart 12 and the master application program 14 by executing step r8 in Fig. 5 described above are received from the first control communication circuit 18 in step u3, and the second processing circuit 27 stores them in the second memory 20. By executing the master application program 14, the second physician can examine the second medical chart 12. As a result of the examination, in the case where the second physician accepts the request for medical care, it is determined whether the acceptance signal 46 is transmitted from the second processing circuit 27 by operating the input-output device 43 in step u4, the second control communication circuit 28 transmits the acceptance signal 46 to the server 3 in step u5. The reception of the acceptance signal 46 in the first control processing device 1 is determined in step r9 of Fig. 5 described above. As a result of the examination of the second medical chart 12 by the second physician, when the second physician does not accept the request of medical care, the acceptance signal 46 is not transmitted, and the process proceeds step u4 to step u12, so that the operation is ended.

After examining the second medical chart 12, when the second physician can diagnose the pet without the additional examination data 16, the second physician does not request the first physician to additionally send the additional examination data 16 in step u6. Thus, the second physician does not transmit the additional request signal 47. In next step u10, the second physician creates and inputs the medical diagnostic report 15 on the basis of the second medical chart 12. In step u11, the second control communication circuit 28 transmits the medical diagnostic report 15, which is received in the first control processing device 1 via the server 3 in step r12 of Fig. 5 described above, whereby the first physician can receive the medical diagnostic report 15.

When the second physician determines that the second medical chart 12 alone is not sufficient for accurate medical diagnosis of the pet, and thus, requests the first physician to additionally send the additional examination data 16 in step u6, the additional request signal 47 for the additional examination data 16 is transmitted in step u7.

This additional request signal 47 is received in the first control communication circuit 18 via the server 3 in step r11 of Fig. 5 described above. The first physician creates the additional examination data 16 requested by the additional request signal 47. In step r17, the third medical chart 13 for the second physician including the second medical chart 12 and also the additional examination data 16 is transmitted by the first control communication circuit 18 together with the master application program 14.

The second control communication circuit 28 determines the reception of the third medical chart 13 and the master application program 14 via the server 3 in step u8, receives them in step u9, and stores them in the second memory 20. The second physician creates the medical diagnostic report 15 on the basis of the third medical chart 13 including the second medical chart 12 and also the additional examination data 16. The medical diagnostic report 15 inputted in step u10 is transmitted from the second control communication circuit 28 in step u11, and is received in step r12 in the first control communication circuit 18 via the server 3, allowing the first physician to procure it. The first physician can provide the advanced medical care by the medical diagnostic report 15 by the second physician.

Fig. 10 is a block diagram illustrating a configuration of a DM issuing device 4. The DM issuing device 4 includes the DM issuance control communication circuit 78, the DM issuance memory 76, and the DM issuing unit 79. The DM issuance control communication circuit 78 receives DM issuance information 49 from the first control communication circuit 18 via the server 3. The DM issuance memory 76 stores the DM issuance information 49 received by the DM issuance control communication circuit 78. The DM issuing unit 79 issues DM to the delivery destination corresponding to the owner for each animal on the expected DM issuance date 67g (Fig. 4L) according to the DM issuance information 49 stored in the DM issuance memory 76.

Fig. 11 is a flowchart subsequent to Fig. 5 for describing operations of the first processing circuit 17 and the first control communication circuit 18 illustrated in Fig. 2. If it is determined in step r4 of Fig. 5 described above that there is an instruction to commission the task of issuing DM by the input-output device 41, the process proceeds to step r20 of Fig. 11, and pet information such as pet name, whether or not the pet is a predetermined DM target, and the like is read from the first memory 10, and customer's name, address, e-mail address, and the like are read out in step r21. In step r22, vaccination histories 67a, 67b (Fig. 4H) including a date for each animal are read. In step r23, medical data histories 51 (Fig. 4A), 67c (Fig. 4E), 67d, 67e (Fig. 4F), 67f (Fig. 4G) including a date for each animal are read.

In step r24, the expected next vaccination date from vaccination histories 67a, 67b (Fig. 4H) including a date, and thus, a corresponding appropriately expected DM issuance date before the expected next vaccination date is determined by calculation by the first processing circuit 17 or the input-output device 41. In addition, regarding medical data histories 51 (Fig. 4A), 67c (Fig. 4E), 67d, 67e (Fig. 4F), 67f (Fig. 4G) including a date, which may be appropriately changed and corrected on the basis of the medical diagnostic report 15, an appropriately expected DM issuance date for medical inquiry about animals is also determined in a similar manner. In step r25, a predetermined text 71a (Fig. 4J) to prompt the owners to allow vaccination and a predetermined text 71b (Fig. 4K) for medical inquiry about animals are determined by calculation by the first processing circuit 17 or the input-output device 41. In this way, in step r26, the DM issuance information 49 of the first memory 10 is created, and in step r27, the first control communication circuit 18 transmits it to the server 3, and the process returns to step r14 of Fig. 5.

Fig. 12 is a flowchart consequent to Fig. 7 for describing the operations of the server processing circuit 37 and the server control communication circuit 38 illustrated in Fig. 2. When it is determined in step s1a of Fig. 7 described above that there is an instruction to commission the task of issuing DM by the input-output device 41, the process proceeds to step s20 of Fig. 12, and the DM issuance information 49 is received, stored in the server memory 30, and transmitted to the DM issuing device 4 in step s21. Then, the process returns to step s5 of Fig. 5.

Fig. 13 is a flowchart for describing the operations of the DM issuance processing circuit 77, the DM issuance control communication circuit 78, and the DM issuing unit 79 of the DM issuing device 4. The process proceeds from step q1 to step q2, and it is determined whether the DM issuance control communication circuit 78 has received the DM issuance information 49. When the DM issuance information 49 has been received, it is stored in the DM issuance memory 76 in step q3. In the step q4, when the commission of the task of issuing the DM is accepted as illustrated in Fig. 4L described above, the confirmation of the commission is returned from the DM issuance control communication circuit 78 to the first control processing device 1 via the server 3. In step q5, the DM issuing unit 79 issues DM 75, for example, as illustrated in Fig. 4J, prints on a postcard 70 by a printer and posts it or transmits it to the terminal device of the owner, for example, as illustrated in Fig. 4K, by e-mail or the like via a communication line 9 such as the Internet.

In another embodiment of the present invention, in place of the configuration of the first control processing device 1 and the DM issuing device 4 via the server 3, the server 3 may be omitted, and the first control processing device 1 and the DM issuing device 4 may be connected via a communication line.

Although the embodiments of the present disclosure have been described in detail above, the present disclosure is not limited to the above-described embodiments, and various changes, improvements, and the like can be made without departing from the gist of the present disclosure. All or part of each of the above-described embodiments can be combined as appropriate without contradiction.

### Reference Signs List

1 first control processing device
2 second control processing device
3 server
4 DM issuing device
5, 6, 7, 9 communication line
8 examination device
10 first memory
11 first medical chart
12 second medical chart
13 third medical chart
14 master application program
15 medical diagnostic report
16 additional examination data
17 first processing circuit
18 first control communication circuit
20 second memory
27 second processing circuit
28 second control communication circuit
30 server memory
37 server processing circuit
38 server control communication circuit
44 request signal
46 acceptance signal
47 additional request signal
49 DM issuance information
51, 67c, 67d, 67e, 67f date and history of medical data
67a, 67b date and history of vaccination
67g expected DM issuance date
68a, 68b photograph
70 postcard
71a, 71b text
72a, 72b name and address
75 direct mail
76 DM issuance memory
78 DM issuance control communication circuit
79 DM issuing unit
A, B, C first veterinary hospital
D, E second veterinary hospital
F commission destination for task of issuing DM

## Claims

1. A remote medical examination device for animals, the remote medical examination device comprising:
(a) a first control processing for a first physician device installed at a first veterinary hospital;
(b) a second control processing device for a second physician installed at a second veterinary hospital; and
(c) a server connected to the first control processing device and the second control processing device via communication lines, wherein:
(d) the first control processing device includes
(d1) a first memory that stores
a first medical chart provided for each of one or more animals to be given medical care, the first medical chart including owner information about an owner of each animal, identification information about each animal, medical data by the first physician, and examination data including an image,
a second medical chart created from the first medical chart with at least part of the owner information excluded, and
a master application program for creating and outputting the first medical chart and the second medical chart,
(d2) a first processing circuit that executes the master application program to create the first medical chart and the second medical chart and store them in the first memory, and
(d3) a first control communication circuit that
outputs, to the server, a request signal about the animal for which medical diagnosis is requested to the second physician in the second veterinary hospital selected by the first physician, the second medical chart of the animal for which the medical diagnosis is requested, and the master application program,
receives from the server an acceptance signal and a medical diagnostic report by the second physician about the animal for which the medical diagnosis is requested and stores them in the first memory;
(e) the server includes
(e1) a server control communication circuit that is connected to the first control communication circuit and the second control processing device via the communication lines,
(e2) a server memory, and
(e3) a server processing circuit that
stores, in the server memory, the request signal, the second medical chart, and the master application program from the first control communication circuit, which have been received by the server control communication circuit, transmits them to the second control processing device by the server control communication circuit,
stores, in the server memory, the acceptance signal and the medical diagnostic report from the second control processing device, which have been received by the server control communication circuit, and transmits them to the first control communication circuit by the server control communication circuit; and
(f) the second control processing device includes
(f1) a second control communication circuit that receives the request signal from the server control communication circuit and, when accepting the medical care request for the animal, transmits the acceptance signal for the animal to the server,
(f2) a second memory that stores the second medical chart and the master application program downloaded from the server for each animal corresponding to the acceptance signal, and
(f3) a second processing circuit that executes the master application program stored in the second memory to create the medical diagnostic report based on the second medical chart by the second physician, and
(f4) the second control communication circuit transmits the medical diagnostic report from the second processing circuit to the server.

2. A remote medical examination method for animals, the remote medical examination method **characterized in that**:
(a) the remote medical examination device for animals according to claim 1 is prepared;
(b) the first physician in the first veterinary hospital selects one or more second physicians in one or more second veterinary hospitals and the animal for which medical diagnosis is requested, and transmits the second medical chart of the animal for which medical diagnosis is requested and the master application program, which have been stored in the first memory of the first control processing device, for the selected second veterinary hospital by the first control communication circuit;
(c) the second physician in the second veterinary hospital accepts the requested medical diagnosis of the animal in the second control processing device, creates the medical diagnostic report based on the second medical chart of the animal for which the medical diagnosis is requested, and transmits the medical diagnostic report for the first veterinary hospital by the second control communication circuit; and
(d) the first physician in the first veterinary hospital determines the medical treatment policy on a basis of the medical diagnostic report that has been created by the second physician and that is downloaded to the first control processing device.

3. A remote medical examination device for animals, the remote medical examination device comprising:
(a) a first control processing device for a first physician installed at a first veterinary hospital;
(b) a second control processing device for a second physician installed at a second veterinary hospital; and
(c) a server connected to the first control processing device and the second control processing device via communication lines, wherein:
(d) the first control processing device includes
(d1) a first memory that stores
a first medical chart provided for each of one or more animals to be given medical care, the first medical chart including owner information about an owner of each animal, identification information about each animal, medical data by the first physician, and examination data including an image,
a second medical chart created from the first medical chart with at least part of the owner information excluded,
additional examination data added by the first physician in response to an additional request signal, and
a master application program for creating and outputting the first medical chart, the second medical chart, and the additional examination data,
(d2) a first processing circuit that executes the master application program to create the first medical chart, the second medical chart, and the additional examination data and store them in the first memory, and
(d3) a first control communication circuit that
outputs, to the server, a request signal about the animal for which medical diagnosis is requested to the second physician in the second veterinary hospital selected by the first physician, the second medical chart of the animal for which the medical diagnosis is requested, the master application program, and the additional examination data corresponding to the additional request signal,
receives from the server an acceptance signal and a medical diagnostic report by the second physician about the animal for which the medical diagnosis is requested and stores them in the first memory;
(e) the server includes
(e1) a server control communication circuit that is connected to the first control communication circuit and the second control processing device via the communication lines,
(e2) a server memory, and
(e3) a server processing circuit that
stores, in the server memory, the request signal, the second medical chart, the additional examination data, and the master application program from the first control communication circuit, which have been received by the server control communication circuit, transmits them to the second control processing device by the server control communication circuit,
stores, in the server memory, the acceptance signal, the additional request signal, and the medical diagnostic report from the second control processing device, which have been received by the server control communication circuit, and transmits them to the first control communication circuit by the server control communication circuit; and
(f) the second control processing device includes
(f1) a second control communication circuit that receives the request signal from the server control communication circuit and, when accepting the medical care request for the animal, transmits the acceptance signal for the animal to the server control communication circuit,
(f2) a second memory that stores the second medical chart, the additional examination data, and the master application program downloaded from the server for each animal corresponding to the acceptance signal, and
(f3) a second processing circuit that executes the master application program stored in the second memory to output the additional request signal that requests the examination data not included in the second medical chart as the additional examination data and to create the medical diagnostic report based on the additional examination data by the second physician, and
(f4) the second control communication circuit transmits the additional request signal and the medical diagnostic report from the second processing circuit to the server.

4. A remote medical examination method for animals, the remote medical examination method **characterized in that**:
(a) the remote medical examination device for animals according to claim 3 is prepared;
(b) the first physician in the first veterinary hospital selects one or more second physicians in one or more second veterinary hospitals and the animal for which medical diagnosis is requested, and transmits the second medical chart of the animal for which medical diagnosis is requested and the master application program, which have been stored in the first memory of the first control processing device, for the selected second veterinary hospital by the first control communication circuit;
(C) the second physician in the second veterinary hospital accepts the requested medical diagnosis of the animal in the second control processing device, outputs, by the second processing circuit, the additional request signal for requesting examination data not included in the second medical chart of the animal, for which medical diagnosis is requested, as the additional examination data to the first physician, and transmits the additional request signal by the second control communication circuit;
(d) the first physician in the first veterinary hospital creates the additional examination data in response to the additional request signal and transmits it for the selected second veterinary hospital by the first control communication circuit;
(e) the second physician in the second veterinary hospital creates the medical diagnostic report based on the additional examination data in the second control processing device and transmits the medical diagnostic report for the first veterinary hospital by the second control communication circuit; and
(f) the first physician in the first veterinary hospital determines a medical treatment policy on a basis of the medical diagnostic report that has been created by the second physician and that has been downloaded to the first control processing device.

5. A remote medical examination device for animals, the remote medical examination device comprising:
(a) a first control processing device for a first physician installed at a first veterinary hospital;
(b) a second control processing device for a second physician installed at a second veterinary hospital; and
(c) a server connected to the first control processing device and the second control processing device via communication lines wherein:
(d) the first control processing device includes
(d1) a first memory that stores
a first medical chart provided for each of one or more animals to be given medical care, the first medical chart including owner information about an owner of each animal, identification information about each animal, medical data by the first physician, and examination data including an image,
a second medical chart created from the first medical chart with at least part of the owner information excluded,
a third medical chart including additional examination data added to the second medical chart by the first physician in response to an additional request signal, and
a master application program for creating and outputting the first to third medical charts,
(d2) a first processing circuit that executes the master application program to create the first to third medical charts and store them in the first memory, and
(d3) a first control communication circuit that
outputs, to the server, a request signal about the animal for which medical diagnosis is requested to the second physician in the second veterinary hospital selected by the first physician, the second medical chart of the animal for which the medical diagnosis is requested, the master application program, and the third medical chart corresponding to the additional request signal,
receives from the server an acceptance signal and a medical diagnostic report by the second physician about the animal for which the medical diagnosis is requested and stores them in the first memory;
(e) the server includes
(e1) a server control communication circuit that is connected to the first control communication circuit and the second control processing device via the communication lines,
(e2) a server memory, and
(e3) a server processing circuit that
stores, in the server memory, the request signal, the second medical chart, the third medical chart, and the master application program from the first control communication circuit, which have been received by the server control communication circuit, transmits them to the second control processing device by the server control communication circuit,
stores, in the server memory, the acceptance signal, the additional request signal, and the medical diagnostic report from the second control processing device, which have been received by the server control communication circuit, and transmits them to the first control communication circuit by the server control communication circuit; and
(f) the second control processing device includes
(f1) a second control communication circuit that receives the request signal from the server control communication circuit and, when accepting the medical care request for the animal, transmits the acceptance signal for the animal to the server control communication circuit,
(f2) a second memory that stores the second medical chart, the third medical chart, and the master application program downloaded from the server for each animal corresponding to the acceptance signal, and
(f3) a second processing circuit that executes the master application program stored in the second memory to output the additional request signal for requesting the examination data not included in the second medical chart as the additional examination data and to create the medical diagnostic report based on the third medical chart by the second physician, and
(f4) the second control communication circuit transmits the additional request signal and the medical diagnostic report from the second processing circuit to the server.

6. A remote medical examination method for animals, the remote medical examination method **characterized in that**:
(a) the remote medical examination device for animals according to claim 5 is prepared;
(b) the first physician in the first veterinary hospital selects one or more second physicians in one or more second veterinary hospitals and the animal for which medical diagnosis is requested, and transmits the second medical chart of the animal for which medical diagnosis is requested and the master application program, which have been stored in the first memory of the first control processing device, for the selected second veterinary hospital by the first control communication circuit;
(c) the second physician in the second veterinary hospital accepts the requested medical diagnosis of the animal in the second control processing device, outputs, by the second processing circuit, the additional request signal for requesting the examination data not included in the second medical chart of the animal, for which medical diagnosis is requested, as the additional examination data to the first physician, and transmits the additional request signal by the second control communication circuit;
(d) the first physician in the first veterinary hospital creates the additional examination data in response to the additional request signal and transmits the third medical chart including the created additional examination data in the second medical chart for the selected second veterinary hospital by the first control communication circuit;
(e) the second physician in the second veterinary hospital creates the medical diagnostic report based on the third medical chart in the second control processing device and transmits the medical diagnostic report for the first veterinary hospital by the second control communication circuit; and
(f) the first physician in the first veterinary hospital determines a medical treatment policy on a basis of the medical diagnostic report that has been created by the second physician and that has been downloaded to the first control processing device.

7. The remote medical examination device for animals according to claim 1, 3, or 5, wherein
the owner information includes at least one of a name and an address of the owner,
the identification information about the animal includes at least one character of a numeral and a symbol to identify the animal,
the medical data includes data about at least one of vaccine, filaria prevention, and flea prevention for medical checkup, medical diagnosis, and medical treatment,
the examination data including an image includes an X-ray photograph of the body of the animal, data on at least one of blood test and blood chemistry screening test, and
the second medical chart does not include the name and address of the owner.

8. A computer program for causing a computer to function as the remote medical examination device for animals according to claim 1, 3, or 5.

9. A recording medium that stores the computer program according to claim 8.

10. A medical examination device for animals that can commission a task of issuing direct mail, the medical examination device **characterized in that**:
(a) a control processing device installed at a veterinary hospital and a DM issuing device installed at a commission destination commissioned with a task of issuing direct mail DM are connected to each other via communication lines;
(b) the control processing device includes
(b1) a first memory that stores
names and addresses that are delivery destinations corresponding to a plurality of animal owners,
vaccination histories including a date for each animal and medical data histories including a date for each animal, and
DM issuance information including a predetermined text to prompt the owners to allow vaccination and a predetermined text for medical inquiry about the animals,
(b2) a first processing circuit that calculates an expected DM issuance date on a basis of the date of vaccination or the date of medical data, creates the DM issuance information including the expected DM issuance date and the texts for vaccination or medical inquiry corresponding to the expected DM issuance date, and stores the DM issuance information in the first memory, and
(b3) a first control communication circuit that transmits the DM issuance information in the first memory to the DM issuing device via the communication lines; and
(c) the DM issuing device includes
(c1) a DM issuance control communication circuit that receives the DM issuance information from the first control communication circuit,
(c2) a DM issuance memory that stores the DM issuance information that has been received by the DM issuance control communication circuit, and
(c3) a DM issuing unit that issues the DM to the delivery destination corresponding to the owner for each animal on the expected DM issuance date according to the DM issuance information stored in the DM issuance memory.

11. A medical examination device for animals that can commission a task of issuing direct mail, the medical examination device comprising:
(a) a first control processing device for a first physician installed at a first veterinary hospital;
(b) a second control processing device for a second physician installed at a second veterinary hospital;
(c) a DM issuing device installed at a commission destination commissioned with the task of issuing direct mail DM; and
(d) a server connected to the first control processing device, the second control processing device, and the DM issuing device via communication lines wherein:
(e) the first control processing device includes:
(e1) a first memory that stores
a first medical chart provided for each of one or more animals to be given medical care, the first medical chart including owner information about an owner of each animal, identification information about each animal, medical data by the first physician, and examination data including an image,
a second medical chart created from the first medical chart with at least part of the owner information excluded, and
a master application program for creating and outputting the first medical chart and the second medical chart, and
that further stores DM issuance information, the DM issuance information including
names and addresses that are delivery destinations corresponding to a plurality of the animal owners,
vaccination histories including a date for each animal and medical data histories including a date for each animal, and
a predetermined text to prompt the owners to allow vaccination and the predetermined text for medical inquiry about the animals,
the first memory further storing a medical diagnostic report from the second control processing device via the server and the vaccination histories and the medical data histories including a date based on the medical diagnostic report after being changed;
(e2) a first processing circuit that executes the master application program to create the first medical chart and the second medical chart and to store them in the first memory; and
(e3) a first control communication circuit that
outputs, to the server, a request signal about the animal for which medical diagnosis is requested to the second physician in the second veterinary hospital selected by the first physician, the second medical chart of the animal for which the medical diagnosis is requested, the master application program, and further the DM issuance information,
receives from the server an acceptance signal and the medical diagnostic report by the second physician about the animal for which the medical diagnosis is requested and stores them in the first memory;
(f) the server includes
(f1) a server control communication circuit that is connected to the first control communication circuit and the second control processing device via the communication lines,
(f2) a server memory, and
(f3) a server processing circuit that
stores, in the server memory, the request signal, the second medical chart, and the master application program from the first control communication circuit, which have been received by the server control communication circuit, transmits them to the second control processing device by the server control communication circuit,
stores, in the server memory, the DM issuance information from the first control communication circuit, which has been received by the server control communication circuit, transmits the DM issuance information to the DM issuing device by the server control communication circuit,
stores, in the server memory, the acceptance signal and the medical diagnostic report from the second control processing device, which have been received by the server control communication circuit, and transmits them to the first control communication circuit by the server control communication circuit; and
(g) the second control processing device includes
(g1) a second control communication circuit that receives the request signal from the server control communication circuit and, when accepting the medical care request for the animal, transmits the acceptance signal for the animal to the server control communication circuit,
(g2) a second memory that stores the second medical chart and the master application program downloaded from the server for each animal corresponding to the acceptance signal, and
(g3) a second processing circuit that executes the master application program stored in the second memory to create the medical diagnostic report based on the second medical chart by the second physician,
(g4) the second control communication circuit transmitting the medical diagnostic report from the second processing circuit to the server; and
(h) the DM issuing device includes
(h1) a DM issuance control communication circuit that receives the DM issuance information from the first control communication circuit via the server control communication circuit,
(h2) a DM issuance memory that stores the DM issuance information that has been received by the DM issuance control communication circuit, and
(h3) a DM issuing unit that issues the DM to the delivery destination corresponding to the owner for each animal on the expected DM issuance date according to the DM issuance information stored in the DM issuance memory.

12. A medical examination device for animals that can commission a task of issuing direct mail, the medical examination device comprising:
(a) a first control processing device for a first physician installed at a first veterinary hospital;
(b) a second control processing device for a second physician installed at a second veterinary hospital;
(c) a DM issuing device installed at a commission destination commissioned with the task of issuing direct mail DM; and
(d) a server connected to the first control processing device, the second control processing device, and the DM issuing device via communication lines, wherein:
(e) the first control processing device includes
(e1) a first memory that stores
a first medical chart provided for each of one or more animals to be given medical care, the first medical chart including owner information about an owner of each animal, identification information about each animal, medical data by the first physician, and examination data including an image,
a second medical chart created from the first medical chart with at least part of the owner information excluded,
additional examination data added by the first physician in response to an additional request signal, and
a master application program for creating and outputting the first medical chart, the second medical chart, and the additional examination data, and
that further stores DM issuance information, the DM issuance information including
names and addresses that are delivery destinations corresponding to a plurality of the animal owners,
vaccination histories including a date for each animal and medical data histories including a date for each animal, and
a predetermined text to prompt the owners to allow vaccination and a predetermined text for medical inquiry about the animals,
the first memory storing a medical diagnostic report from the second control processing device via the server and vaccination histories and medical data histories including a date based on the medical diagnostic report after being changed,
(e2) a first processing circuit that executes the master application program to create the first medical chart, the second medical chart, and the additional examination data and to store them in the first memory, and
(e3) a first control communication circuit that
outputs, to the server, a request signal about the animal for which medical diagnosis is requested to the second physician in the second veterinary hospital selected by the first physician, the second medical chart of the animal for which the medical diagnosis is requested, the master application program, the additional examination data in response to the additional request signal, and further the DM issuance information,
receives from the server an acceptance signal and the medical diagnostic report by the second physician about the animal for which the medical diagnosis is requested and stores them in the first memory;
(f) the server includes
(f1) a server control communication circuit that is connected to the first control communication circuit and the second control processing device via the communication lines,
(f2) a server memory, and
(f3) a server processing circuit that
stores, in the server memory, the request signal, the second medical chart, the additional examination data, and the master application program from the first control communication circuit, which have been received by the server control communication circuit, transmits them to the second control processing device by the server control communication circuit, and
further stores, in the server memory, the DM issuance information from the first control communication circuit, which has been received by the server control communication circuit, transmits the DM issuance information to the DM issuing device by the server control communication circuit,
stores, in the server memory, the acceptance signal, the additional request signal, and the medical diagnostic report from the second control processing device, which have been received by the server control communication circuit, and transmits them to the first control communication circuit by the server control communication circuit; and
(g) the second control processing device includes
(g1) a second control communication circuit that receives the request signal from the server control communication circuit and, when accepting the medical care request for the animal, transmits the acceptance signal for the animal to the server control communication circuit,
(g2) a second memory that stores the second medical chart, the additional examination data, and the master application program downloaded from the server for each animal corresponding to the acceptance signal, and
(g3) a second processing circuit that executes the master application program stored in the second memory to output the additional request signal for requesting the examination data not included in the second medical chart as the additional examination data and to create the medical diagnostic report based on the additional examination data by the second physician;
(g4) the second control communication circuit transmits the additional request signal and the medical diagnostic report from the second processing circuit to the server; and
(h) the DM issuing device includes
(h1) a DM issuance control communication circuit that receives the DM issuance information from the first control communication circuit via the server control communication circuit,
(h2) a DM issuance memory that stores the DM issuance information that has been received by the DM issuance control communication circuit, and
(h3) a DM issuing unit that issues the DM to the sending destination of the owner for each animal on the expected DM issuance date according to the DM issuance information stored in the DM issuance memory.
